# EUROPEAN PATENT SPECIFICATION

(11) **EP 3 356 561 B1**
(45) Date of publication and mention of the grant of the patent: **27.10.2021**
(21) Application number: 16852729.9
(22) Date of filing: 30.09.2016
(51) Int. Cl.: C12Q 1/6883

(54) **THERAPEUTIC AND DIAGNOSTIC METHODS FOR AUTOIMMUNE DISEASES AND/OR INFLAMMATION**
THERAPEUTISCHE UND DIAGNOSTISCHE VERFAHREN FÜR AUTOIMMUNKRANKHEITEN UND/ODER ENTZÜNDUNGEN
PROCÉDÉS THÉRAPEUTIQUES ET DIAGNOSTIQUES POUR DES MALADIES AUTOIMMUNES ET/OU DES INFLAMMATIONS

(30) Priority: 02.10.2015 US 201562236514 P
(43) Date of publication of application: 08.08.2018
(73) Proprietor: Momenta Pharmaceuticals, Inc., Cambridge, MA 02142 (US)
(72) Inventor: WASHBURN, Nathaniel, Cambridge, MA 02142 (US); HALVEY, Patrick, Cambridge, MA 02142 (US); MCCONNELL, Kevin, Cambridge, MA 02142 (US); FARUTIN, Victor, Cambridge, MA 02142 (US); CAPILA, Ishan, Cambridge, MA 02142 (US); LING, Leona, E., Cambridge, MA 02142 (US); MANNING, Anthony, Cambridge, MA 02142 (US)
(74) Representative: Mathys & Squire
(86) International application number: PCT/US2016/054833
(87) International publication number: WO 2017/059276

(56) References cited:
- WO-A1-2015/054290
- WO-A2-03/035904
- WO-A2-2011/088219
- WO-A2-2011/088219
- Kazumitsu Tomita ET AL: "Association between tumor necrosis factor-alpha and Fc-gamma receptor polymorphisms with infliximab in Crohn's disease", Hepato-gastroenterology, 1 May 2010 (2010-05-01), page 535, XP055586205, Greece Retrieved from the Internet: URL:http://eolit-p.internal.epo.org/api/or ders/581228/pdf
- CAITLIN GILLIS ET AL: "Contribution of Human Fc[gamma]Rs to Disease with Evidence from Human Polymorphisms and Transgenic Animal Studies", FRONTIERS IN IMMUNOLOGY, vol. 5, 30 May 2014 (2014-05-30), XP055404150, DOI: 10.3389/fimmu.2014.00254
- KENZIE D. MACISAAC ET AL: "Pre-Treatment Whole Blood Gene Expression Is Associated with 14-Week Response Assessed by Dynamic Contrast Enhanced Magnetic Resonance Imaging in Infliximab-Treated Rheumatoid Arthritis Patients", PLOS ONE, vol. 9, no. 12, 12 December 2014 (2014-12-12), page e113937, XP055586173, DOI: 10.1371/journal.pone.0113937
- HUGHES, LB ET AL.: 'Genetic Risk Factors for Infection in Patients with Early Rheumatoid Arthritis.' GENES AND IMMUNITY. vol. 5, no. 8, December 2004, pages 641 - 647, XP055383782
- SAMMARITANO, LR ET AL.: 'Anti-Cardiolipin IgG Subclasses- Association of IgG2 with Arterial and/or Venous Thrombosis.' ARTHRITIS AND RHEUMATISM. vol. 40, no. 11, November 1997, pages 1998 - 2006, XP055383788
- IIDA, S ET AL.: 'The N-linked oligosaccharide at FcyRIIIa Asn-45: An inhibitory element for high FcyRIIIa binding affinity to IgG Glycoforms Lacking Core Fucosylation.' GLYCOBIOLOGY. vol. 19, no. 2, 24 October 2008, pages 126 - 134, XP055332309
- LEJEUNE, J ET AL.: 'FCGR2C Genotyping by Pyrosequencing Reveals Linkage Disequilibrium with FCGR3A V158F and FCGR2A H131R Polymorphisms in a Caucasian Population.' MABS. vol. 4, no. 6, 02 October 2012, pages 784 - 787, XP055383794

## Description

### BACKGROUND OF THE INVENTION

Anti-tumor necrosis factor-alpha (TNF-a) agents are a first-line biologic therapy in a number of autoimmune diseases. However, one third or more of the patients who initiate anti-TNFa therapy for the first time do not respond. There is a need for treatment methods for patients with autoimmune diseases and inflammations, as well as diagnostics to predict the responsiveness and non-responsiveness of patients to anti-TNFa therapies.

Tomita, et al. (Hepato-Gastroenterology, 57:535-539, 2010) describe an association between TNFα and FcγRIIIB polymorphisms with infliximab in treatment of Crohn's disease. Gillis, et al. (Frontiers in Immunology, vol 5, article 254, 2014) describe associations between FcyR polymorphisms and human pathologies.

### SUMMARY OF THE INVENTION

The present disclosure relates generally to methods of treating a subject having an autoimmune disease, and/or having inflammation, e.g., inflammation related to activated neutrophils. These methods include the steps of (a) determining one or more of: (i) the genotype of the gene encoding Fcγ receptor (FcyR) IIIb (FcγRIIIb), (ii) the glycophenotype of FcγRIIIb, (iii) the genotype of the gene encoding FcγRIIa, and/or (iv) the genotype of the gene encoding FcyRllc, in a biological sample of the subject, and (b) administering to the subject an agent other than an anti-TNFa agent (e.g., an Fc-activity modulating agent, an anti-CTLA4 agent, an anti-CD20 agent, or an anti-IL6 agent) if at least (i) one or two alleles of FcyRlllb NA1 is present in the biological sample, (ii) the FcyRlllb NA1 glycophenotype is present in the biological sample, (iii) one or two allele of FcγRIIa H131 is present in the biological sample, and/or (iv) one or two allele of FcγRIIc variant rs61801826 (genotype FCGR2C Chr1:161569198 registered in the NCBI dbSNP as ID rs61801826) is present in the biological sample. In some instances of the present disclosure, the subject does not receive an anti-TNFa agent in addition to the Fc-activity modulating agent. In other instances, the subject is administered an anti-TNFa agent in combination with the Fc-activity modulating agent. The disclosure also relates to methods of determining the responsiveness or non-responsiveness of a subject to an anti-TNFa treatment based on the genotype of the gene encoding one or more of FcγRIIIb, FcγRIIa,FcyRllc and/or the glycophenotype of FcγRIIIb.

In particular, the present invention provides a method of predicting the responsiveness of a patient to an anti-TNFa treatment, said method comprising: (a) identifying a patient having inflammation or an autoimmune disease; (b) determining the presence of the FcyRllc in a biological sample from said patient, and (c) selecting the patient for treatment with an anti-TNFa agent if the patient lacks the FcyRllc variant rs61801826. The following embodiments relating to the administering of an anti-TNFalpha agent and to the methods of treatment with such agents are not according to the invention and are present for illustration purposes only. In one embodiment, the administered agent is an anti-CTLA agent (e.g., abatacept), an anti-IL6 agent (e.g., tocilizumab), or an anti-CD20 agent (e.g., rituximab).

In one embodiment, the administered agent is an Fc-activity modulating agent (e.g., a selective immunomodulator of Fc receptor (SIF), intravenous immunoglobulins (IVIg), or a stradomer).

In one aspect, disclosed herein is a method of treating a subject having an autoimmune disease and/or inflammation includes the steps of: (a) determining the genotype of the gene encoding Fcγ receptor IIIb (FcγRIIIb) and/or the glycophenotype of FcγRIIIb in a biological sample from the subject; and (b) administering an Fc-activity modulating agent to the subject if one or two alleles of FcyRlllb NA1 and/or the FcyRlllb NA1 glycophenotype is present in the biological sample of the subject. In some instances step (a) includes the step of determining both the genotype of the gene encoding FcγRIIIb and the glycophenotype of FcyRlllb in a biological sample from the subject. In some instances, the subject is administered the Fc-activity modulating agent if two alleles of FcγRIIIb NA1 and/or the glycophenotype of FcγRIIIb is present in the biological sample of the subject.

In another aspect, the disclosure features a method of treating a subject having an autoimmune disease and/or inflammation by administering an Fc-activity modulating agent to the subject if the subject is identified as having at least one allele of FcyRlllb NA1, the FcyRlllb NA1 glycophenotype, and/or one or two allele of FcyRllc variant rs61801826.

In another aspect, the disclosure features a method of treating a subject having an autoimmune disease and/or inflammation by administering an Fc-activity modulating agent to the subject if the subject is identified as having at least one allele of FcyRlllb NA1 and/or the FcyRlllb NA1 glycophenotype.

In another aspect, the disclosure features a method of treating a subject having an autoimmune disease and/or inflammation by administering an Fc-activity modulating agent to the subject if the subject is identified as having two alleles of FcyRlllb NA1 and/or the FcyRlllb NA1 glycophenotype.

In another aspect, the disclosure features a method of treating a subject having an autoimmune disease and/or inflammation by administering an Fc-activity modulating agent to the subject if the subject is identified as having at least one allele of FcyRlllb NA1 and/orthe FcγRIIIb NA1 glycophenotype and at least one allele of FcγRIIa H131.

In another aspect, the disclosure features a method of treating a subject having an autoimmune disease and/or inflammation by administering an Fc-activity modulating agent to the subject if the subject is identified as having (a) one or two alleles of FcyRlllb NA1, the FcyRlllb NA1 glycophenotype, and/or one or two allele of FcyRllc variant rs61801826; and (b) one or two alleles of FcyRlla H131.

In some instances, the method includes administering an Fc-activity modulating agent to the subject if the subject is identified as having two alleles of FcyRlllb NA1 and/orthe FcyRlllb NA1 glycophenotype and at least one allele of FcyRlla H131.

In some instances, the method includes administering an Fc-activity modulating agent to the subject if the subject is identified as having at least one allele of FcyRlllb NA1 and/orthe FcγRIIIb NA1 glycophenotype and two alleles of FcyRlla H131.

In some instances, the method includes administering an Fc-activity modulating agent to the subject if the subject is identified as having the FcyRlllb NA1 glycophenotype, at least one allele of FcyRlla H131, and the FcyRllc variant rs61801826.

In some instances, the method includes administering an Fc-activity modulating agent to the subject if the subject is identified as having at least one allele of FcγRIIIb NA1 and/or the FcyRlllb NA1 glycophenotype, two alleles of FcyRlla H131, and the FcyRllc variant rs61801826.

In some instances, the method includes administering an Fc-activity modulating agent to the subject if the subject is identified as having two alleles of FcyRlllb NA1 and/orthe FcyRlllb NA1 glycophenotype and two alleles of FcyRlla H131.

In some instances, the method includes administering an Fc-activity modulating agent to the subject if the subject is identified as having two alleles of FcyRlllb NA1 and/or the FcyRlllb NA1 glycophenotype, two alleles of FcyRlla H131, and the FcyRllc variant rs61801826.

In another aspect, disclosed is a method of treating a subject having an autoimmune disease and/or inflammation includes the steps of: (a) determining the genotype of the FcγRIIc gene in a biological sample from the subject and (b) administering an Fc-activity modulating agent if one or two alleles of FcγRIIc variant rs61801826 is present in the biological sample of the subject. In one instance, the FcγRIIc variant rs61801826 is homozygous. In certain instances, an Fc-activity modulating agent is administered if the subject has one or more FcγRIIc variants shown in Table 8 as being associated with non-response, in addition to, or instead of, FcγRIIc variant rs61801826.

In some instances of either foregoing aspect, the determining step (a) further includes determining the genotype of the gene encoding FcyRlla in a biological sample from the subject. The subject is administered the Fc-activity modulating agent if one or two alleles of FcyRlla H131 is also present in the biological sample of the subject in addition to one or two alleles of FcyRlllb NA1, the FcyRlllb NA1 glycophenotype, and/or one or two alleles of FcγRIIc variant rs61801826.

In some instances, the subject is administered the Fc-activity modulating agent if two alleles of FcyRlla H131 are also present in the biological sample of the subject in addition to one or two alleles of FcyRlllb NA1, the FcyRlllb NA1 glycophenotype, and/or one or two alleles of FcγRIIc variant rs61801826.

In some instances, the determining step (a) includes determining the presence or absence of the genotype or glycophenotype of FcγRIIIb and the FcγRIIc genotype in a biological sample from the subject. The subject is administered the Fc-activity modulating agent if one or two alleles of FcγRIIc variant rs61801826 are present in the biological sample of the subject in addition to one or two alleles of FcyRlllb NA1 and/or the FcyRlllb NA1 glycophenotype.

In some instances, the subject in any of the aforementioned methods of the disclosure has been previously treated with an anti-TNFa agent for the autoimmune disease and/or inflammation. In some instances, the subject in any of the aforementioned methods of the disclosure has not been previously treated with an anti-TNFa agent for the autoimmune disease and/or inflammation.
In some instances, the method further includes administering to the subject a biologic therapy that is not an anti-TNFa agent in combination with the Fc-activity modulating agent. In some instances, the biologic therapy is selected from the group consisting of an anti-CTLA agent (e.g., abatacept), an anti-IL6 agent (e.g., tocilizumab), and an anti-CD20 agent (e.g., rituximab).

In some instances, the subject is also administered an anti-TNFa agent in combination with the Fc-activity modulating agent. In some instances, the subject is not also administered an anti-TNFa agent.

In some instances of any of the aforementioned methods, the Fc-activity modulating agent may be an Fc-containing polypeptide or an antibody that specifically targets an Fc receptor (e.g., FcyRlllb or FcγRIIc). Fc-activity modulating agents specifically exclude anti-TNFa agents. An Fc-containing polypeptide can be selected from the group consisting of: selective immunomodulators of Fc receptors (SIFs), intravenous immunoglobulins (IVIg), and stradomers. Fc-containing polypeptides specifically exclude anti-TNFa agents.

In some instances, the SIF is SIF3. SIF3 contains three Fc domains constructed from four polypeptides. The first polypeptide has the formula A-L-B, in which A includes a first Fc domain monomer, L is a linker, and B includes a second Fc domain monomer. The second polypeptide has the formula A'-L'-B', in which A' includes a third Fc domain monomer, L' is a linker, and B' includes a fourth Fc domain monomer. The third polypeptide includes a fifth Fc domain monomer, while the fourth polypeptide includes a sixth Fc domain monomer. A of the first polypeptide and A' of the second polypeptide combine to form a first Fc domain, B of the first polypeptide and the fifth Fc domain monomer combine to form a second Fc domain, and B' of the second polypeptide and the sixth Fc domain monomer combine to form a third Fc domain.

In some instances, the IVIg is hypersialylated IVIg. In some instances, at least 50% (e.g., 60%, 70%, 80%, 82%, 85%, 87%, 90%, 92%, 94%, 95%, 97%, 98% up to and including 100%) of branched glycans on the Fc domain of the IgGs are di-sialylated by way of NeuAc-α2,6-Gal terminal linkages. In some instances, less than 50% (e.g., less than 40%, 30%, 20%, 15%, 10%, 5%, 4%, 3%, 2%, 1%) of branched glycans on the Fc domain of the IgGs are mono-sialylated (e.g., on the α1,3 arm or the α1,6 arm) by way of a NeuAc-α2,6-Gal terminal linkage. In other instances, at least 50% (e.g., 60%, 70%, 80%, 82%, 85%, 87%, 90%, 92%, 94%, 95%, 97%, 98% up to and including 100%) of branched glycans on the Fc domain of the IgGs are di-sialylated by way of NeuAc-α2,6-Gal terminal linkages and less than 50% (e.g., less than 40%, 30%, 20%, 10%, 15%, 5%, 4%, 3%, 2%, 1%) of branched glycans on the Fc domain are mono-sialylated on the α1,3 arm by way of a NeuAc-α2,6-Gal terminal linkage. In other instances, at least 50% (e.g., 60%, 70%, 80%, 82%, 85%, 87%, 90%, 92%, 94%, 95%, 97%, 98% up to and including 100%) of branched glycans on the Fc domain of the IgGs are di-sialylated by way of NeuAc-α2,6-Gal terminal linkages and less than 50% (e.g., less than 40%, 30%, 20%, 10%, 5%, 4%, 3%, 2%, 1%) of branched glycans on the Fc domain of the IgGs are mono-sialylated on the α1,6 arm by way of a NeuAc-α2,6-Gal terminal linkage.

In some instances of any of the aforementioned methods, the subject is also administered a biologic therapy that is not an anti-TNFa agent. In some instances, the biologic therapy is of an anti-CTLA agent (e.g., abatacept), an anti-IL6 agent (e.g., tocilizumab), or an anti-CD20 agent.

In another aspect, disclosed herein is a method of predicting the non-responsiveness of a subject to an anti-TNFa treatment, including determining (i) the genotype of the gene encoding FcγRIIIb in a biological sample from the subject and/or (ii) the glycophenotype of the FcγRIIIb from a biological sample from the subject, wherein the presence of at least one allele of FcγRIIIb NA1 and/or the FcγRIIIb NA1 glycophenotype in a biological sample is indicative that the subject may not respond to the anti-TNFa treatment. The following embodiments relating to the prediction of response to an anti-TNFalpha treatment which do not involve the detection of rs61801826 are not according to the invention and are present for illustration purposes only.

In some instances, the presence of two alleles of FcγRIIIb NA1 is indicative that the subject may not respond to the anti-TNFa treatment. In some instances, the method further includes determining the genotype of the gene encoding FcγRIIa,wherein the further presence of at least one allele of FcyRlla H131 is indicative that the subject may not respond to the anti-TNFa treatment. In some instances, the further presence of two alleles of FcγRIIa H131 is indicative that the subject may not respond to the anti-TNFα treatment.

In another aspect, the disclosure features a method of treating a subject having one or more of rheumatoid arthritis (RA), juvenile RA, polyarticular-course juvenile RA, juvenile idiopathic arthritis, psoriasis, psoriatic arthritis, Crohn's Disease, ulcerative colitis, ankylosing spondylitis, and plague psoriasis, including: (a) determining (i) the genotype of the gene encoding FcγRIIIb in a biological sample from the subject and (ii) the glycophenotype of FcγRIIIb from a biological sample from the subject, and (b) administering to the subject an Fc-activity modulating agent if at least one allele of FcγRIIIb NA1 and the FcyRlllb NA1 glycophenotype is present in the biological sample.

In some instances, the method further includes administering to the subject a biologic therapy that is not an anti-TNFa agent if at least one allele of FcγRIIIb NA1 and the FcγRIIIb NA1 glycophenotype is present in the biological sample. In some instances, the biologic therapy is an anti-CTLA agent (e.g., abatacept), an anti-IL6 agent (e.g., tocilizumab), or an anti-CD20 agent.

In another aspect, the disclosure features a method of predicting the responsiveness of a patient to an anti-TNFa treatment. The method includes the steps of: (a) identifying a patient having inflammation or an autoimmune disease; (b) determining (i) the genotype of the gene encoding FcγRIIIb in a biological sample from the patient and/or (ii) the glycophenotype of the FcγRIIIb from a biological sample from the subject, and (c) selecting the patient for treatment with an Fc-activity modulating agent if the patient has at least one allele of FcyRlllb NA1 and/or the FcyRlllb NA1 glycophenotype.

The invention features a method of predicting the responsiveness of a patient to an anti-TNFa treatment. The method includes the steps of: (a) identifying a patient having inflammation or an autoimmune disease; (b) determining the FcγRIIc genotype in a biological sample from the subject, and (c) selecting the patient for treatment with an anti-TNFa agent if the patient lacks the FcyRllc variant rs61801826.

In another aspect, the disclosure features a method for approving payment of treatment of a subject having an autoimmune disease and/or inflammation in advance of the treatment being performed. This method includes (a) receiving from a service provider a report identifying the FcyRlllb genotype and/or glycophenotype of the subject; and (b) approving payment of treatment of the subject with an Fc-activity modulating agent only if the subject has at least one allele of FcγRIIIb NA1 and/or the FcyRlllb NA1 glycophenotype.

In another aspect, the disclosure features a method for approving payment of treatment of a subject having an autoimmune disease and/or inflammation. This method includes the steps of: (a) receiving from a service provider (i) a report identifying the FcyRlllb genotype and/or glycophenotype of the subject; and (ii) optionally a proposal to treat the subject with an Fc-activity modulating agent; and (b) approving payment of treatment of the subject (e.g., transmitting approval of payment of treatment to a health care provider for the subject) with an Fc-activity modulating agent only if the subject has at least one allele of FcγRIIIb NA1 and/or the FcyRlllb NA1 glycophenotype.

In some instances, steps (a) and (b) are both performed prior to the subject being treated.

In another aspect, the disclosure features a method of treating a subject having an autoimmune disease and/or inflammation, including: (a) determining one or more of: (i) the genotype of the gene encoding Fcγ receptor IIIb (FcyRIIIb), (ii) the glycophenotype of FcγRIIIb, (iii) the genotype of the gene encoding FcyRlla, and/or (iv) the genotype of the gene encoding FcyRllc, in a biological sample of the subject, and (b) administering to the subject an agent other than an anti-TNFa agent (e.g., an Fc-activity modulating agent, an anti-CTLA4 agent, an anti-CD20 agent or an anti-IL6 agent) if one or more of: (i) one or two allele of FcyRlllb NA1 is present in the biological sample, (ii) the FcyRlllb NA1 glycophenotype is present in the biological sample, (iii) one or two allele of FcyRlla H131 is present in the biological sample, or (iv) one or two allele of FcyRllc variant rs61801826 is present in the biological sample.

In another aspect, the disclosure features a method for authorizing treatment of a subject having an autoimmune disease and/or inflammation in advance of the treatment being performed, including (a) receiving in a print or digital media from a service provider a report identifying one or more of: (i) the genotype of the gene encoding Fcγ receptor (FcyR) IIIb (FcyRIIIb), (ii) the glycophenotype of FcγRIIIb, (iii) the genotype of the gene encoding FcγRIIa,or (iv) the genotype of the gene encoding FcyRllc, in a biological sample of the subject, and (b) transmitting in a print or digital media approval of treatment to a health care provider for the subject if one or more of: (i) one or two allele of FcyRlllb NA1 is present in the biological sample, (ii) the FcyRlllb NA1 glycophenotype is present in the biological sample, (iii) one or two allele of FcyRlla H131 is present in the biological sample, or (iv) one or two allele of FcyRllc variant rs61801826 is present in the biological sample.

In another aspect, the disclosure features a method of treating a subject having an autoimmune disease and/or inflammation, the method including: (a) determining the genotype of a subject's gene encoding an Fcγ receptor listed in Table 8, (b) determining the predicted response or non-response of the subject to an anti-TNFa agent based on the determined genotype, according to Table 8, and (c) administering to the subject an anti-TNF agent if the determined genotype correlates with response according to Table 8 OR administering to the subject an agent other than an anti-TNF agent if the determined genotype correlates with non-response according to Table 8.

In another aspect, the disclosure features a method of predicting the responsiveness of a subject to an anti-TNFa treatment using an antibody specific for biantennary sialylated N162 or an antibody specific for aglycosylated N45 in FcyRlllb to detect the presence of biantennary sialylated N162 or aglycosylated N45 in a biological sample from a subject having an autoimmune disease and/or inflammation, wherein the presence of biantennary sialylated N162 or aglycosylated N45 in the biological sample is indicative that the subject may not respond to the anti-TNFa treatment.

In another aspect, the disclosure features a method of predicting the responsiveness of a subject to an anti-TNFa treatment using an antibody specific for triantennary sialylated N162 or an antibody specific for a high mannose glycan on N45 to detect the presence of triantennary sialylated N162 or a high mannose glycan on N45 in a biological sample from a subject having an autoimmune disease and/or inflammation, wherein the presence of triantennary sialylated N162 or a high mannose glycan on N45 in the biological sample is indicative that the subject may respond to the anti-TNFa treatment.

In some instances, the method of using an antibody to predict the responsiveness or responsiveness of a subject to an anti-TNFa treatment further includes using a reagent composition including a labeled antibody specific to the glycan antibody, wherein the reagent composition is capable of providing a detectable signal in the presence of the labeled antibody.

In another aspect, the disclosure features a method of treating a subject having an autoimmune disease and/or inflammation by (a) determining the genotype of the gene encoding FcγRIIIb and/or the glycophenotype of FcγRIIIb in a biological sample from the subject; and (b) administering an anti-TNFa agent to the subject if at least one allele of FcγRIIIb NA2 and/or the FcγRIIIb NA2 glycophenotype is present in the biological sample of the subject.

In some instances, the subject is administered the anti-TNFa agent if two alleles of FcyRlllb NA2 and/or the FcyRlllb NA2 glycophenotype is present in the biological sample of the subject.

In some instances, the determining step (a) further includes determining the genotype of the gene encoding FcyRlla in a biological sample from the subject, wherein the subject is administered the anti-TNFα agent if one allele of FcyRlla R131 is also present in the biological sample of the subject in addition to one or two alleles of FcyRlllb NA2 and/or the FcyRlllb NA2 glycophenotype.

In some instances, the subject is administered the anti-TNFa agent if two alleles of FcyRlla R131 are also present in the biological sample of the subject in addition to one or two alleles of FcyRlllb NA2 and/or the FcyRlllb NA2 glycophenotype.

In some embodiments, the determining step (a) further includes determining the presence or absence of the FcyRllc variant rs61801826 in a biological sample from the subject, wherein the subject is administered the anti-TNFa agent if the FcyRllc variant rs61801826 is absent in the biological sample in addition to one or two alleles of FcyRlllb NA2 and/or the FcyRlllb NA2 glycophenotype being present.

In another aspect, the disclosure features a method of treating a subject having an autoimmune disease and/or inflammation by (a) determining the FcyRllc genotype in a biological sample from the subject; and (b) administering an anti-TNFa agent to the subject if the FcγRIIc variant rs61801826 is absent in the biological sample of the subject. In some instances, the subject is administered the anti-TNFα agent if two alleles of FcyRlla R131 are also present in the biological sample of the subject in addition to the FcyRllc variant rs61801826 being absent. In certain instances, an anti-TNFa agent is administered if the subject has one or more FcyRllc variants shown in Table 8 as being associated with response, in addition to, or instead of an absent FcyRllc variant rs61801826.

In another aspect, the disclosure features a method of treating a subject having an autoimmune disease and/or inflammation by administering an anti-TNFa agent to the subject, wherein the subject is identified as (a) having at least one allele of FcγRIIIb NA2 and/or the FcγRIIIb NA2 glycophenotype and/or (b) lacking the FcγRIIc variant rs61801826.

In another aspect, the disclosure features a method of treating a subject having an autoimmune disease and/or inflammation by administering an anti-TNFa agent to the subject, wherein the subject is identified as having two alleles of FcyRlllb NA2 and/or the FcγRIIIb NA2 glycophenotype.

In another aspect, the disclosure features a method of treating a subject having an autoimmune disease and/or inflammation by administering an anti-TNFa agent to the subject if the subject is identified as having at least one allele of FcyRlllb NA2 and/or the FcyRlllb NA2 glycophenotype and at least one allele of FcyRlla R131.

In some instances, the method includes administering an anti-TNFa agent to the subject if the subject is identified as having two alleles of FcyRlllb NA2 and/or the FcγRIIIb NA2 glycophenotype and at least one allele of FcyRlla R131.

In some instances, the method includes administering an anti-TNFa agent to the subject if the subject is identified as having at least one allele of FcyRlllb NA2 and/or the FcyRlllb NA2 glycophenotype and two alleles of FcγRIIa R131.

In some instances, the method includes administering an anti-TNFa agent to the subject if the subject is identified as having two alleles of FcyRlllb NA2 and/or the FcγRIIIb NA2 glycophenotype and two alleles of FcyRlla R131.

In some instances, the method includes administering an anti-TNFa agent to the subject if the subject is identified as lacking the FcγRIIc hapalotype.

In another aspect, the disclosure features a method of predicting the responsiveness of a patient to an anti-TNFa treatment. The method includes the steps of: (a) identifying a patient having inflammation or an autoimmune disease; (b) determining (i) the genotype of the gene encoding FcyRlllb in a biological sample from the patient and/or (ii) the glycophenotype of the FcγRIIIb from a biological sample from the subject, and (c) selecting the patient for treatment with an anti-TNFa agent if the patient has at least one allele of FcγRIIIb NA2 and/or the FcγRIIIb NA2 glycophenotype.

In another aspect, the disclosure features a method for approving payment of treatment of a subject having an autoimmune disease and/or inflammation, including (a) receiving from a service provider a report identifying the FcyRlllb genotype and/or glycophenotype of the subject; and (b) approving payment of treatment of the subject with an anti-TNFa agent only if the subject has at least one allele of FcγRIIIb NA2 and/or the FcγRIIIb NA2 glycophenotype.

In another aspect, the disclosure features a method for approving payment of treatment of a subject having an autoimmune disease and/or inflammation. This method includes the steps of: (a) receiving from a service provider (i) a report identifying the FcγRIIIb genotype and/or glycophenotype of the subject; and (ii) optionally a proposal to treat the subject with an Fc-activity modulating agent; and (b) approving payment of treatment of the subject (e.g., transmitting approval of payment of treatment to a health care provider for the subject) with an anti-TNFa agent only if the subject has at least one allele of FcγRIIIb NA2 and/or the FcγRIIIb NA2 glycophenotype.

In some instances, steps (a) and (b) are both performed prior to the subject being treated.

In some embodiments or instances of any of the aforementioned methods, the autoimmune disease is a tumor necrosis factor alpha (TNF-a) mediated disease. In some embodiments or instances of any of the aforementioned methods, the autoimmune disease is rheumatoid arthritis (RA), juvenile RA, polyarticular-course juvenile RA, juvenile idiopathic arthritis, psoriasis, psoriatic arthritis, Crohn's Disease, ulcerative colitis, ankylosing spondylitis, plaque psoriasis, multiple sclerosis, systemic lupus erythematosus, myasthenia gravis, juvenile onset diabetes, glomerulonephritis, autoimmune thyroiditis, Behcet's disease, graft rejection, graft-versus-host disease, Kawasaki's disease, sarcoidosis, pyoderma gangrenosum, depression, bronchial asthma, diabetes mellitus, malignancies, septic shock, bullous dermatitis, neutrophilic dermatitis, toxic epidermal necrolysis, systemic vasculitis, pyoderma gangrenosum, pustular dermatitis, alcoholic hepatitis, cerebral malaria, hemolytic uremic syndrome, pre-eclampsia, allograft rejection, uveitis, otitis media, snakebite, erythema nodosum, myelodysplastic syndromes, dermatomyositis, polymyositis, immune reconstitution inflammatory syndrome of AIDS patients, systemic sclerosis, IgG4-related disease, or alopecia areata. In some embodiments or instances of any of the aforementioned methods, the inflammation is myocarditis.

In some embodiments or instances of any of the aforementioned methods, the anti-TNFa agent is selected from the group consisting of adalimumab, infliximab, etanercept, certolizumab pegol, and golimumab.
In some embodiments or instances of the methods, the subject is treated with Orencia®, tociluzimab, Rituxan®, or anakinra.

In some embodiments or instances of the methods, the subject is treated with methotrexate, prednisone, hydroxychloroquine, leflunomide, azathioprine, cyclosporine, tofacitinib, cuprimine, auranofin, or minocycline in combination with the Fc-activity modulating agent and/or anti-TNFa agent.

In another aspect, the disclosure features a method of predicting the responsiveness of a subject to an anti-TNFa treatment. The method includes determining (i) the genotype of the gene encoding FcγRIIIb in a biological sample from the subject and/or (ii) the glycophenotype of the FcγRIIIb from a biological sample from the subject, wherein the presence of at least one allele of FcγRIIIb NA1 and/or the FcγRIIIb NA1 glycophenotype in a biological sample is indicative that the subject may not respond to the anti-TNFa treatment.

In some instances, the presence of two alleles of FcγRIIIb NA1 and/or the FcγRIIIb NA1 glycophenotype is indicative that the subject may not respond to the anti-TNFa treatment.

In some instances, the method further includes determining the genotype of the gene encoding FcγRIIa,wherein the further presence of at least one allele of FcyRlla H131 in the biological sample from the subject in addition to one or two alleles of FcγRIIIb NA1 and/or the FcγRIIIb NA1 glycophenotype is indicative that the subject may not respond to the anti-TNFa treatment.

In some instances, the method further includes determining the genotype of the gene encoding FcγRIIa,wherein the further presence of two alleles of FcyRlla H131 in the biological sample from the subject in addition to one or two alleles of FcγRIIIb NA1 and/or the FcγRIIIb NA1 glycophenotype is indicative that the subject may not respond to the anti-TNFa treatment.

In some instances, the method further includes determining the presence or absence of the FcyRllc variant rs61801826, wherein the presence of the FcyRllc variant rs61801826 in the biological sample from the subject in addition to one or two alleles of FcγRIIIb NA1 and/or the FcγRIIIb NA1 glycophenotype is indicative that the subject may not respond to the anti-TNFa treatment.

In another aspect, the disclosure features a method of treating a subject having one or more of rheumatoid arthritis (RA), juvenile RA, polyarticular-course juvenile RA, juvenile idiopathic arthritis, psoriasis, psoriatic arthritis, Crohn's Disease, ulcerative colitis, ankylosing spondylitis, and plague psoriasis, including: (a) determining the presence of the FcyRllc variant rs61801826 in a biological sample from the subject, and (b) administering to the subject an Fc-activity modulating agent if the FcyRllc variant rs61801826 is present in the biological sample.

In some instances, the method further includes administering to the subject a biologic therapy that is not an anti-TNFa agent if the FcyRllc variant rs61801826 is present in the biological sample. In some instances, the biologic therapy is an anti-CTLA agent (e.g., abatacept), an anti-IL6 agent (e.g., tocilizumab), or an anti-CD20 agent.

In another aspect, the disclosure features a method of predicting the responsiveness of a subject to an anti-TNFa treatment. The method includes the step of detecting the presence of a nucleotide sequence that encodes FcγRIIIb NA1, or a fragment thereof, in a biological sample from a subject having an autoimmune disease and/or inflammation, wherein the presence of the nucleotide sequence in the biological sample is indicative that the subject may not respond to the anti-TNFa treatment.

In another aspect, the disclosure features a method of predicting the responsiveness of a subject to an anti-TNFa treatment. The method includes the step of detecting the presence of a nucleotide sequence that encodes FcγRIIIb NA2, or a fragment thereof, in a biological sample from a subject having an autoimmune disease and/or inflammation, wherein the presence of the nucleotide sequence in the biological sample is indicative that the subject may respond to the anti-TNFa treatment.

In some embodiments or instances of any of the forgoing aspects, the presence of a genotype is determined by a method that uses allele-specific oligonucleotides, primer extension, allele-specific ligation, sequencing, electrophoretic separation, a 5'-nuclease assay, a template-directed dye-terminator assay, a molecular beacon allele-specific oligonucleotide assay, or a single-base extension assay. In some embodiments or instances, the assay is performed by first reverse-transcribing the target RNA, and then amplifying the resulting cDNA or by using a combined high-temperature reverse-transcription-polymerase chain reaction (RT-PCR).

In some embodiments or instances, the biological sample is a body fluid (e.g., whole blood), a body tissue (e.g., a lung sample), a nasal sample (e.g., a nasal swab or nasal polyps), or sputum. Body fluids include, e.g., whole fresh blood, frozen whole blood, plasma, peripheral blood mononuclear cells, urine, saliva, lymph, sera, semen, synovial fluid, and spinal fluid.

In some embodiments or instances of any of the forgoing aspects, the presence of a glycophenotype is determined by a method using fluorescence-activated cell sorting (FACS), immunoprecipitation, or differential mobility on SDS-PAGE. In some instances, a sandwich ELISA assay is used to capture FcγRIIIb from a cell lysate using an anti-FcγRIIIb antibody, followed by detection by another anti-CD16 (FcγRIII) antibody (e.g., labeled with a fluor or detected with a labeled secondary antibody). Alternatively a reverse sandwich ELISA could be employed, capturing with the non-interfering anti-CD16 antibody and detecting with an antibody specific for FcRlllb.

In some instances, the NA1 and/or NA2 FcyRlllb glycophenotype is determined by a lectin microtiter ELISA. In some instances, the glycophenotype is determined by a method employing enzymatic, chromatographic, mass spectrometry (MS), chromatographic followed by MS, electrophoretic methods, electrophoretic methods followed by MS, or nuclear magnetic resonance (NMR) method.

In some embodiments or instances of any of the above aspects or embodiments, the inflammation is inflammation related to activated neutrophils.

### Definitions

As used herein, the term "genotype" refers to the DNA sequence making up a set of alleles at a particular genetic locus. A genotype can be a description of all or a portion (e.g., the presence or absence of a SNP) of the DNA sequence of one or both the alleles of a gene contained in a subject or a sample (e.g., a biological sample from a subject). The genotype of two or more closely linked loci on a chromosome can describe a haplotype: a set of DNA variations, or polymorphisms, that tend to be inherited together, e.g., a combination of alleles or a set of single nucleotide polymorphisms (SNPs) found on the same chromosome. In the context of this invention, no distinction is made between the genotype of a subject and the genotype of a sample (e.g., a biological sample) from the subject. Although typically a genotype is determined from samples of diploid cells, a genotype can be determined from a sample of haploid cells, such as a sperm cell.

As used herein, the term "polymorphism" refers to the occurrence of two or more genetically determined alternative sequences of a gene in a population. Polymorphisms often occur as a result of changes in one or more specific nucleotides in a genetic sequence of a gene, which lead to alternative sequences of a gene. Typically, the first identified allelic form is arbitrarily designated as the reference form and other allelic forms are designated as alternative or variant alleles. A "common" allele is an allele that is prevalent in a given population (e.g., greater than about 2% of the population carries the allele). For example, FcγRIIa has two polymorphic variants, FcγRIIa R131 and FcγRIIa H131, each encoded by one of the two alleles of FcγRIIa. The amino acid residue at position 131 is either R or H depending on a specific nucleotide (C or T) in the genetic sequence of FcγRIIa.

As used herein, the term "single nucleotide polymorphism" or "SNP" refers to a site of one nucleotide that varies between alleles. Single nucleotide polymorphisms (SNPs) may occur at any region of the gene. In some instances the polymorphism can result in a change in protein sequence. The change in protein sequence may affect protein function. Some polymorphic variants of proteins differ as a result of one or more SNPs. For example, a SNP, nucleotide C or T, in the genetic sequence of FcγRIIa leads to two polymorphic variants of FcγRIIa: FcγRIIa R131 and FcγRIIa H131.

As used herein, the term "glycophenotype" refers to the glycosylation profile of a protein. The glycosylation profile is the collective state or pattern of glycosylation at certain amino acid residues, e.g., asparagine, in a protein. The glycosylation state or pattern at a specific amino acid residue in a protein may affect the protein's folding, function, and/or interaction with another protein. The glycophenotype of a protein sometimes depends on the polymorphic variant of the protein. For example, polymorphic variants FcγRIIIb NA1 and NA2 display different glycophenotypes. FcγRIIIb NA2 is more likely to have a high mannose glycan at position N45 and a triantennary sialylated glycan at position N162. This glycosylation profile of NA2 is also referred to as the FcγRIIIb NA2 glycophenotype. FcγRIIIb NA1 is more likely to have an aglycosylated N45 and a biantennary sialylated glycan at position N162. This glycosylation pattern of NA1 is also referred to as the FcγRIIIb NA1 glycophenotype.

As used herein, the term "glycan" refers to a sugar, which can be a monomer or polymer of sugar residues, such as at least three sugars, and can be linear or branched. A glycan can include natural sugar residues (e.g., glucose, N-acetylglucosamine, N-acetyl neuraminic acid, galactose, mannose, fucose, hexose, arabinose, ribose, xylose, etc.) and/or modified sugars (e.g., 2'-fluororibose, 2'-deoxyribose, phosphomannose, 6'-sulfo N-acetylglucosamine, etc.). The term "glycan" includes homo and heteropolymers of sugar residues. The term "glycan" also encompasses a glycan component of a glycoconjugate (e.g., of a polypeptide, glycolipid, proteoglycan, etc.). The term also encompasses free glycans, including glycans that have been cleaved or otherwise released from a glycoconjugate.

As used herein, the term "high mannose glycan" refers to an oligosaccharide containing 5-9 mannose residues linked to a chitobiose core.

As used herein, the term "biantennary sialylated" or "biantennary sialylation" refers to the sialylation of glycan compositions (i.e., level of branched glycans that are sialylated on an α1,3 arm and/or an α1,6 arm), in which two branches of the glycans are sialylated on an α1,3 arm and/or an α1,6 arm.

As used herein, the term "triantennary sialylated" or "triantennary sialylation" refers to the sialylation of glycan compositions (i.e., level of branched glycans that are sialylated on an α1,3 arm and/or an α1,6 arm), in which three branches of the glycans are sialylated on an α1,3 arm and/or an α1,6 arm.

As used herein, the term "Fc-activity modulating agent" refers to a molecule, e.g., a small molecule or a macromolecule such as a polypeptide (such as an Fc-containing polypeptide or an antibody), that is capable of interfering with or modulating the activity of a wild-type, non-synthetic Fc-domain containing protein or polypeptide (which is not the Fc-containing polypeptide defined herein) or a receptor thereto. For example, the Fc-activity modulating agent may bind to the same target Fc receptor (e.g., FcγRIIIb) as that of the wild-type, non-synthetic Fc-domain containing protein or polypeptide, and thus inhibit the activity of the wild-type, non-synthetic Fc-domain containing protein or polypeptide. Specifically excluded from the definition of "Fc-activity modulating agent" are anti-TNFa agents.

As used herein, the term "Fc-containing polypeptide" refers to associated polypeptide chains forming at least one (e.g., 1-10) Fc domain as described herein. Fc-containing polypeptides can include Fc domain monomers that have the same or different sequences. For example, an Fc-containing polypeptide can have two Fc domains, one of which includes IgG1 or IgG1-derived Fc domain monomers, and a second which includes IgG2 or IgG2-derived Fc domain monomers. In the present invention, an Fc domain does not include any variable region of an antibody, e.g., V_{H}, V_{L}, CDR, or HVR. An Fc domain forms the minimum structure that binds to an Fc receptor, e.g., FcγRI, FcγRIIa,FcγRIIb, FcγRIIc, FcγRIIIa, FcγRIIIb, or FcyRIV. In the present invention, an Fc-containing polypeptide is selected from the group consisting of selective immunomodulators of Fc receptors (SIFs), intravenous immunoglobulins (IVIg), and stradomers. Specifically excluded from the definition of "Fc-containing polypeptide" are anti-TNFα agents.

As used herein, the term "selective immunomodulators of Fc receptors" or "SIFs" refers to engineered IgG Fc constructs described in US Patent Publication No. 20160229913.

As used herein, the term "IVIg" refers to intravenous immunoglobulin (e.g., Gammagard®, Privigen® or Octagam®) as well as recombinant IgG immunoglobulin.

A "stradomer" is a biomimetic compound capable of binding two or more Fcγ receptors and having one of four different physical conformations: serial, cluster, core or Fc fragment, as described in U.S. Patent No. 8,680,237 at column 18, line 1, to column 19, line 6.

As used herein, the term "biologic therapy" refers to a protein therapy that is not an anti-TNFa agent. Biologic therapies include antibodies such as abatacept, tocilizumab, and rituximab.

As used herein, the term "tumor necrosis factor alpha (TNF-a) mediated disease" refers to a disease or disorder involving the cytokine TNF-α. TNF-α mediated diseases include, but are not limited to, rheumatoid arthritis (RA), juvenile RA, polyarticular-course juvenile RA, juvenile idiopathic arthritis, psoriasis, psoriatic arthritis, Crohn's Disease, ulcerative colitis, ankylosing spondylitis, plague psoriasis, multiple sclerosis, systemic lupus erythematosus, myasthenia gravis, juvenile onset diabetes, glomerulonephritis, autoimmune thyroiditis, Behcet's disease, graft rejection, and graft-versus-host disease, Kawasaki's disease, sarcoidosis, pyoderma gangrenosum, depression, bronchial asthma, diabetes mellitus, malignancies, septic shock, bullous dermatitis, neutrophilic dermatitis, toxic epidermal necrolysis, systemic vasculitis, pyoderma gangrenosum, pustular dermatitis, alcoholic hepatitis, cerebral malaria, hemolytic uremic syndrome, pre-eclampsia, allograft rejection, uveitis, otitis media, snakebite, erythema nodosum, myelodysplastic syndromes, dermatomyositis, polymyositis, immune reconstitution inflammatory syndrome of AIDS patients, systemic sclerosis, IgG4-related disease, and alopecia areata. These diseases and disorders are sometimes treated by using an anti-TNFa agent, which targets TNF-α and/or one or both of its receptors TNFR1 and TNFR2.

As used herein, the term "anti-TNFa agent" refers to an agent, e.g., a protein or small molecule drug, that suppresses the physiological response (e.g., an inflammatory response) caused by TNF-α in a TNF-α mediated disease by binding and inhibiting TNF-α or by binding and inhibiting one or both of the receptors of TNF-α, e.g., TNFR1 and/or TNFR2. Anti-TNFa agents include, but are not limited to, adalimumab, infliximab, etanercept, certolizumab pegol, and golimumab.

As used herein, the term "responsiveness" refers to the responsiveness to treatment or therapy, e.g., an anti-TNFa treatment, of a subject, as determined by a recognized standard of measurement. For example, in rheumatoid arthritis, responsiveness to a treatment may be analyzed and measured using 28-joint Disease Activity Score (DAS28), 28-joint Disease Activity Score using C-reactive protein value (DAS28 CRP score), joint assessment (e.g., tender joint count, swollen joint count), pain measures (e.g., 10-cm visual analogue scale (VAS), 5-point categorical scale, Health Assessment Questionnaire (HAQ) pain index, and Arthritis Impact Measurement Scales (AIMS)), and quality-of-life measures (e.g., Problem Elicitation Technique (PET)) (see, e.g., Buchbinder et al. Arthritis Rheum. 38:1568-80, 1995), as well as guidelines published by the European League Against Rheumatism (EULAR) and/or American College of Rheumatology (ACR) (see, e.g., Felson et al. Arthritis & Rheu. 63:573-586, 2011; Felson et al. Arthritis & Rheu. 38:727-35, 1995).

As used herein, the term "subject" is a human, such as a human patient.

As used herein, the term "biological sample" refers to any biological sample obtained from a subject including body fluids, body tissue (e.g., lung samples), nasal samples (including nasal swabs or nasal polyps), sputum, cells, or other sources. Body fluids include, e.g., whole fresh blood, frozen whole blood, plasma (including fresh or frozen), peripheral blood mononuclear cells, urine, saliva, lymph, sera, semen, synovial fluid, and spinal fluid. Methods for obtaining tissue biopsies and body fluids from mammals are well known in the art.

As used herein, the term "polynucleotide" (or "nucleotide sequence" or "nucleic acid molecule") refers to an oligonucleotide, nucleotide, or polynucleotide, and fragments or portions thereof, and to DNA or RNA of genomic or synthetic origin, which may be single- or double- stranded, and represent the sense or anti-sense strand.

As used herein, the term "polypeptide" describes a single polymer in which the monomers are amino acid residues which are joined together through amide bonds. A polypeptide is intended to encompass any amino acid sequence, either naturally occurring, recombinant, or synthetically produced.

As used herein, the term "pharmaceutical composition" refers to a medicinal or pharmaceutical formulation that contains an active ingredient as well as excipients and diluents to enable the active ingredient suitable for the method of administration.

As used herein, the term "pharmaceutically acceptable carrier" refers to an excipient or diluent in a pharmaceutical composition. The pharmaceutically acceptable carrier must be compatible with the other ingredients of the formulation and not deleterious to the recipient. The nature of the carrier differs with the mode of administration. For example, for oral administration, a solid carrier is preferred; for intravenous administration, an aqueous solution carrier is generally used.

As used herein, the term "therapeutically effective amount" refers to an amount, e.g., pharmaceutical dose, effective in inducing a desired biological effect in a subject or patient or in treating a patient having a disease or disorder described herein. It is also to be understood herein that a "therapeutically effective amount" may be interpreted as an amount giving a desired therapeutic effect, either taken in one dose or in any dosage or route, taken alone or in combination with other therapeutic agents.

As used herein, the term "kit" is any manufacture (e.g., a package or container) including at least one composition (e.g., a composition including an Fc-activity modulating agent), and instruction for administering the composition in combination with one or more additional therapeutic agents (e.g., an anti-TNFa agent or a biologic therapy that is not an anti-TNFa agent).

### DESCRIPTION OF THE DRAWINGS

**FIG. 1** is an amino acid sequence alignment of the sequences of FcγRIIIb NA2, FcγRIIIb NA1, FcγRIIIb SH, and FcγRIIIa.
FIG. 2 is a schematic that shows haploptype-specific glycosylation at N45 and N162 of FcγRIIIb NA2 and FcγRIIIb NA1.
**FIG.** 3 are three graphs that show the correlations between responders/non-responders and the relative abundance of glycosylation at N162 of soluble FcγRIIIb (sFcγRIIIb) (left), the correlations between the haplotype of sFcγRIIIb from subjects having RA and the relative abundance of glycosylation at N162 of sFcγRIIIb (middle), and the correlations between the haplotype of sFcγRIIIb from the healthy donors and the relative abundance of glycosylation at N162 of sFcγRIIIb (right).
**FIG.** 4 is a graph that shows the change in disease severity with treatment (measured by the change in DAS28CRP score) in subjects who are FcγRIIIb NA2/NA2 homozygous or NA1/NA1 homozygous.
**FIG.** 5 is a graph that shows the correlations between responders/non-responders who are FcγRIIIb NA1/NA2 heterozygous (NA1/NA2) and the relative abundance of aglycosylation at N45 of FcγRIIIb.
**FIG.** 6 is a graph that shows the change in disease severity with treatment (measured by the change in DAS28CRP score) in subjects categorized by FcγRIIIb and FcγRIIa genotype.
**FIG.** 7 is a graph that shows the change in disease severity with treatment (measured by the change in DAS28CRP score) in subjects categorized by FcγRIIIb, FcγRIIa and FcγRIIc genotype.
**FIG.** 8 is a graph that shows the change in disease severity with treatment (measured by the change in DAS28CRP score) in subjects who are FcγRIIIb NA2/NA2 homozygous or NA1/NA1 homozygous.
**FIG.** 9 is a graph that shows the change in disease severity with treatment (measured by the change in DAS28CRP score) in subjects categorized by FcγRIIIb and FcγRIIa genotype.

### DETAILED DESCRIPTION OF THE INVENTION

### I. Therapeutic Methods

The present disclosure generally includes methods of diagnosing and treating a subject having an autoimmune disease (e.g., a TNF-α mediated disease), and/or inflammation. The treatment or approval of treatment of the subject is informed by the subject's genotype of the gene encoding one or more of FcγRIIIb, FcγRIIa,and FcyRllc and/or the glycophenotype of FcγRIIIb. It has been found that certain Fc receptor genotypes or glycophenotypes as described herein associate with patient response or non-response to anti-TNF agents. Thus, patients having the genotypes or glycophenotypes described herein as being associated with response, are identified as candidates for treatment with an anti-TNFa agent (e.g., adalimumab, infliximab, etanercept, certolizumab pegol, and golimumab). Patients having the genotypes or glycophenotypes described herein as being associated with non-response to anti-TNF agents, are identified as candidates for treatment with (a) Fc-activity modulating agents such as: selective immunomodulators of Fc receptors (SIFs), intravenous immunoglobulins (IVIg), stradomers, (b) an Fc-containing polypeptide or antibody that specifically targets an Fc receptor (e.g., targets FcγRIIIb), or (c) another non-anti-TNFa agent (e.g., a biologic non-anti-TNFa agent) for the treatment of an autoimmune disease or inflammation, such as an anti-CTLA agent (e.g., abatacept), an anti-IL6 agent (e.g., tocilizumab), or an anti-CD20 agent (e.g., rituximab).

In some instances, the subject patient has been previously treated with an anti-TNFa agent for the autoimmune disease and/or inflammation before being administered an Fc-activity modulating agent or other non-anti-TNFa agent for the treatment of the disease. In some instances, the subject patient has not been previously treated with an anti-TNFa agent before being administered an Fc-activity modulating agent or other non-anti-TNFa agent for the treatment of the disease. In some instances, the method further includes administering to the subject a biologic therapy that is not an anti-TNFa agent in combination with the Fc-activity modulating agent. In some instances, the biologic therapy is an anti-CTLA agent (e.g., abatacept), an anti-IL6 agent (e.g., tocilizumab), or an anti-CD20 agent.

In some instances, the subject is also administered an anti-TNFa agent in combination with the Fc-activity modulating agent. In some instances, the subject is not administered an anti-TNFa agent in combination with the Fc-activity modulating agent.

In some instances of any of the described methods, the Fc-activity modulating agent is an Fc-containing polypeptide or an antibody that specifically targets an Fc receptor (e.g., FcγRIIIb). Examples of Fc-containing polypeptides are selective immunomodulators of Fc receptors (SIFs), intravenous immunoglobulins (IVIg), and stradomers.

In any of the described methods of the invention or disclosure, the autoimmune disease is a tumor necrosis factor alpha (TNF-a) mediated disease. TNF-α mediated diseases include rheumatoid arthritis (RA), juvenile RA, polyarticular-course juvenile RA, juvenile idiopathic arthritis, psoriasis, psoriatic arthritis, Crohn's Disease, ulcerative colitis, ankylosing spondylitis, plaque psoriasis, multiple sclerosis, systemic lupus erythematosus, myasthenia gravis, juvenile onset diabetes, glomerulonephritis, autoimmune thyroiditis, Behcet's disease, graft rejection, graft-versus-host disease, Kawasaki's disease, sarcoidosis, pyoderma gangrenosum, depression, bronchial asthma, diabetes mellitus, malignancies, septic shock, bullous dermatitis, neutrophilic dermatitis, toxic epidermal necrolysis, systemic vasculitis, pyoderma gangrenosum, pustular dermatitis, alcoholic hepatitis, cerebral malaria, hemolytic uremic syndrome, pre-eclampsia, allograft rejection, uveitis, otitis media, snakebite, erythema nodosum, myelodysplastic syndromes, dermatomyositis, polymyositis, immune reconstitution inflammatory syndrome of AIDS patients, systemic sclerosis, IgG4-related disease, and alopecia areata.

In any of the described methods of the invention or disclosure, the inflammation can be myocarditis.

In any of the described methods of the invention or disclosure, the anti-TNFa agent can be, e.g., selected from the group consisting of adalimumab, infliximab, etanercept, certolizumab pegol, and golimumab. In some embodiments or instances, the subject is treated with methotrexate, prednisone, hydroxychloroquine, leflunomide, azathioprine, cyclosporine, tofacitinib, cuprimine, auranofin, or minocycline in combination with the Fc-activity modulating agent and/or anti-TNFa agent.

The genotypes of the genes encoding FcγRIIIb and FcyRlla can be determined using any of the methods or assays described herein (e.g., in Section IV of the Detailed Description of the Invention) or that are known in the art. The glycophenotypes of FcγRIIIb NA1 and NA2 can also be determined using the methods described herein (e.g., in Section VII of the Detailed Description of the Invention) or that are known in the art. The methods of treatment of the disclosure are administered by any suitable means. Pharmaceutical compositions, routes, and dosage of administration suitable for the methods of the disclosure are described in detail further herein.

### II. Diagnostic Methods

Some methods of the disclosure include determining the genotype or glycophenotype of a subject for predicting the responsiveness or non-responsiveness of a subject to an anti-TNFa treatment. The methods are useful in assessing, choosing, and implementing the appropriate treatment methods and therapies for a subject having an autoimmune disease, and/or inflammation. For example, a subject identified as having an autoimmune disease and/or inflammation provides a biological sample (e.g., a blood sample) before treatment and the sample is examined to determine the genotype of the genes encoding FcγRIIIb, FcyRlla and/or FcγRIIc, and/or the glycophenotype of FcγRIIIb, to determine whether the subject is likely to be responsive or non-responsive to an anti-TNFa treatment.

As described herein, the FcγRIIIb NA1genotype or glycophenotype, the FcyRlla H131 genotype, and the FcyRllc variant rs61801826 are associated with negative response to anti-TNF therapy. As described herein, the FcγRIIIb NA2genotype or glycophenotype, the FcγRIIa R131 genotype, and the absence of the FcyRllc variant rs61801826 are associated with positive response to anti-TNF therapy.

Methods of genotyping useful in the diagnostic methods will detect heterozygous or homozygous variants and polymorphisms. In the methods described herein, the genotype for one, two or three of the genes encoding FcγRIIIb, FcyRlla and/or FcyRllc may be determined.

The genotype of a sample or subject for a particular gene can be determined in a number of ways (see also Sec. IV below). Typically, methods of genotyping include restriction fragment length polymorphism identification (RFLPI) of genomic DNA, random amplified polymorphic detection (RAPD) of genomic DNA, amplified fragment length polymorphism detection (AFLPD), polymerase chain reaction (PCR), DNA sequencing, allele specific oligonucleotide (ASO) probes, and hybridization to DNA microarrays or beads.

The glycophenotypes of a subject or sample can be determined in various ways, for example, by way of methods using antibodies. For example, one may use an antibody specific for biantennary sialylated N162 or an antibody specific for aglycosylated N45 in FcyRlllb to detect the presence of biantennary sialylated N162 or aglycosylated N45 in a biological sample from a subject having an autoimmune disease and/or inflammation, wherein the presence of biantennary sialylated N162 or aglycosylated N45 in the biological sample is indicative that the subject may not respond to the anti-TNFa treatment. One may use an antibody specific for triantennary sialylated N162 or an antibody specific for a high mannose glycan on N45 to detect the presence of triantennary sialylated N162 or a high mannose glycan on N45 in a biological sample from a subject having an autoimmune disease and/or inflammation, wherein the presence of triantennary sialylated N162 or a high mannose glycan on N45 in the biological sample is indicative that the subject may respond to the anti-TNFa treatment.

In some methods, one may use a reagent composition including a labeled antibody specific to the detection antibody, wherein the reagent composition is capable of providing a detectable signal in the presence of the labeled antibody.

### III. Fc-activity Modulating Agents

The Fc-activity modulating agent used in the methods of the disclosure may be an Fc-containing polypeptide or an antibody that specifically targets an Fc receptor (e.g., FcγRIIIb). An Fc-containing polypeptide refers to associated polypeptide chains forming at least one (e.g., 1-10) Fc domain as described herein. Fc-containing polypeptides can include Fc domain monomers that have the same or different sequences. For example, an Fc-containing polypeptide can have two Fc domains, one of which includes IgG1 or IgG1-derived Fc domain monomers, and a second which includes IgG2 or IgG2-derived Fc domain monomers. In the present disclosure, an Fc domain does not include any variable region of an antibody, e.g., V_{H}, V_{L}, CDR, or HVR. An Fc domain forms the minimum structure that binds to an Fc receptor, e.g., FcyRI, FcγRIIa,FcγRIIb, FcγRIIIa, FcγRIIIb, FcyRIV. Examples of Fc-containing polypeptides are selective immunomodulators of Fc receptors (SIFs), intravenous immunoglobulins (IVIg), and stradomers.

### Selective immunomodulators of Fc receptors (SIFs)

SIFs refer to engineered IgG Fc constructs described in US 20160229913. SIFs include at a minimum, one functional Fc domain formed from a dimer of two Fc domain monomers. SIFs may include 2-10 Fc domains, e.g., engineered Fc constructs having 2, 3, 4, 5, 6, 7, 8, 9, or 10 Fc domains. SIFs have greater binding affinity and/or avidity than a single wild-type Fc domain for an Fc receptor, e.g., FcγRIIIb.

A SIF includes Fc domain monomers, with pairs of Fc domain monomers forming Fc domains. In some instances, an Fc domain monomer includes an IgG hinge domain, an IgG C_{H}2 antibody constant domain, and an IgG C_{H}3 antibody constant domain. A SIF may include 2-6 (e.g., 2, 3, 4, 5, 6) associated polypeptides, each polypeptide including at least one Fc domain monomer, wherein each Fc domain monomer of the SIF is the same or differs by no more than 10 amino acids, e.g., no more than 8, 6, 4, or 2 amino acids. SIFs do not include an antigen-binding domain such as a Fab found in an antibody.

In some instances, the SIF is SIF3. SIF3 contains three Fc domains constructed from four polypeptides. The first polypeptide has the formula A-L-B, in which A includes a first Fc domain monomer, L is a linker, and B includes a second Fc domain monomer. The second polypeptide has the formula A'-L'-B', in which A' includes a third Fc domain monomer, L' is a linker, and B' includes a fourth Fc domain monomer. The third polypeptide includes a fifth Fc domain monomer, while the fourth polypeptide includes a sixth Fc domain monomer. A of the first polypeptide and A' of the second polypeptide combine to form a first Fc domain, B of the first polypeptide and the fifth Fc domain monomer combine to form a second Fc domain, and B' of the second polypeptide and the sixth Fc domain monomer combine to form a third Fc domain.

An Fc domain monomer of a SIF can include engineered amino acid substitutions at the interface of two interacting C_{H}3 antibody constant domains such that two Fc domain monomers of an Fc domain selectively form a dimer with each other, thus preventing the formation of uncontrolled multimers. The engineered amino acid substitutions make favorable the dimerization of the two C_{H}3 antibody constant domains as a result of the compatibility of amino acids chosen for those substitutions. The ultimate formation of the favored Fc domain is selective over other Fc domains which form from Fc domain monomers lacking the engineered amino acid substitutions or with incompatible amino acid substitutions. As described in US Patent Publication No. 20160229913, SIFs can include engineered cavities, engineered protuberances, and positively- or negatively-charged amino acids.

### Intravenous immunoglobulins (IVIg)

Fc-containing polypeptides that are used in the methods of the disclosure also include IVIg. IVIg includes intravenous immunoglobulin and recombinant IgG. As is well-known in the art, intravenous immunoglobulin is a blood product that contains the pooled, polyvalent IgG immunoglobulins from the plasma of a large number of (typically >1000) blood donors. Intravenous immunoglobulin typically contains substantially (i.e., more than 95%) unmodified IgG, which has Fc-dependent effector functions. Commercially available IVIg products include Gamimune N®, Gammagard®, Gammar®, and Sandoglobulin®.

Recombinant IgG refers to IgG immunoglobulins that are made through genetic engineering using techniques such as recombinant DNA technology and gene splicing. Recombinant IgG may be produced in vitro from a cell line (e.g., a mammalian cell line, e.g., a human cell line) and purified using conventional protein purification techniques in the art (e.g., protein A/G affinity column chromatography, size-exclusion column chromatography, and ion exchange column chromatography). For example, a recombinant human IgG may be constructed from the IgG sequence of a non-human organism (e.g., a mouse) by way of codon optimization. In some instances, recombinant IgG may include amino acid substitutions, additions, and/or deletions in the Fc domain of the IgG that, e.g., lead to a change in effector function, e.g., decreased complement-dependent cytolysis (CDC), antibody-dependent cell-mediated cytolysis (ADCC), and/or antibody-dependent cell-mediated phagocytosis (ADCP), and/or decreased B-cell killing.

In some instances, the IVIg is hypersialylated IVIg (e.g., as is described in US Patent Publication No. 20160257754. In some instances, at least 50% (e.g., 60%, 70%, 80%, 82%, 85%, 87%, 90%, 92%, 94%, 95%, 97%, 98% up to and including 100%) of branched glycans on the Fc domain of the IgGs are di-sialylated by way of NeuAc-α2,6-Gal terminal linkages. In some instances, less than 50% (e.g., less than 40%, 30%, 20%, 15%, 10%, 5%, 4%, 3%, 2%, 1%) of branched glycans on the Fc domain of the IgGs are mono-sialylated (e.g., on the α1,3 arm or the α1,6 arm) by way of a NeuAc-α2,6-Gal terminal linkage. In other instances, at least 50% (e.g., 60%, 70%, 80%, 82%, 85%, 87%, 90%, 92%, 94%, 95%, 97%, 98% up to and including 100%) of branched glycans on the Fc domain of the IgGs are di-sialylated by way of NeuAc-α2,6-Gal terminal linkages and less than 50% (e.g., less than 40%, 30%, 20%, 10%, 15%, 5%, 4%, 3%, 2%, 1%) of branched glycans on the Fc domain are mono-sialylated on the α1,3 arm by way of a NeuAc-α2,6-Gal terminal linkage. In other instances, at least 50% (e.g., 60%, 70%, 80%, 82%, 85%, 87%, 90%, 92%, 94%, 95%, 97%, 98% up to and including 100%) of branched glycans on the Fc domain of the IgGs are di-sialylated by way of NeuAc-α2,6-Gal terminal linkages and less than 50% (e.g., less than 40%, 30%, 20%, 10%, 5%, 4%, 3%, 2%, 1%) of branched glycans on the Fc domain of the IgGs are mono-sialylated on the α1,6 arm by way of a NeuAc-α2,6-Gal terminal linkage.

### Stradomers

Stradomers refer to biomimetic compounds capable of binding to two or more Fcγ receptors. Stradomers are described in, e.g., U.S. Patent No.: 8,680,237. A stradomer can have four different physical conformations: serial, cluster, core, or Fc fragment.

### IV. Detection of Nucleic Acid Polymorphisms

The genotype of a gene (e.g., the gene encoding FcγRIIIb in a subject suffering from an autoimmune disease and/or inflammation) can be determined using detection methods that are known in the art. Most assays entail one of several general protocols: hybridization using allele-specific oligonucleotides, primer extension, allele-specific ligation, sequencing, or electrophoretic separation techniques, e.g., single-stranded conformational polymorphism (SSCP) and heteroduplex analysis. Exemplary assays include 5'-nuclease assays, template-directed dye-terminator incorporation, molecular beacon allele-specific oligonucleotide assays, single-base extension assays, and SNP scoring by realtime pyrophosphate sequences. Analysis of amplified sequences can be performed using various technologies such as microchips, fluorescence polarization assays, and MALDI-TOF (matrix assisted laser desorption ionization-time of flight) mass spectrometry.

Determination of the presence or absence of a particular allele is generally performed by analyzing a nucleic acid sample that is obtained from the subject to be analyzed. Often, the nucleic acid sample includes genomic DNA. The genomic DNA is typically obtained from blood samples, but may also be obtained from other cells or tissues. It is also possible to analyze RNA samples for the presence of polymorphic alleles. For example, mRNA can be used to determine the genotype of a gene at one or more polymorphic sites. In this case, the nucleic acid sample is obtained from cells in which the target nucleic acid is expressed. Such an analysis can be performed by first reverse-transcribing the target RNA using, for example, a viral reverse transcriptase, and then amplifying the resulting cDNA; or using a combined high-temperature reverse-transcription-polymerase chain reaction (RT-PCR).
For determination of genotypes, subject samples, such as those containing cells, or nucleic acids produced by these cells, may be used in the methods of the present disclosure. A biological sample may be taken from a subject having an autoimmune disease (e.g., a TNF-α mediated disease) and/or inflammation. Biological samples useful as samples in the present disclosure include, e.g., body fluids (e.g., whole blood), body tissue (e.g., lung samples), nasal samples (including nasal swabs or nasal polyps), sputum, cells, or other sources. Body fluids include, e.g., whole fresh blood, frozen whole blood, plasma (including fresh or frozen), peripheral blood mononuclear cells, urine, saliva, lymph, sera, semen, synovial fluid, and spinal fluid. Methods for obtaining tissue biopsies and body fluids from mammals are well known in the art.

A method for analysis of biological samples to determine genotypes of the genes encoding FcγRIIIb and FcyRlla is described below. In this method, targeted resequencing is used to isolate and sequence genomic regions of interest within a sample library of DNA. Targeted resequencing allows for the identification of genetic variants in the Fc receptors, including the FcRlllb NA1/NA2 polymorphism. In one embodiment or instance, to perform targeted resequencing, genomic DNA is extracted from cells isolated from human whole blood samples using the QIAmp DNA Mini Kit (Qiagen). This DNA is used to build a sample library. Next, Illumina DesignStudio software is used to design custom oligomer probes targeting the coding regions and the 3' and 5' untranslated regions of one or more Fc receptor genes (FCGR1A, FCGR2A, FCGR2B, FCGR2C, FCGR3A, and FCGR3B). Each probe pair is specific to a 450 base pair sequence, known as an amplicon, of the Fc receptor gene. Each amplicon overlaps its neighboring amplicon by approximately 25 base pairs on both ends, which allows for maximum coverage of the targeted region. The Illumina TruSeq Custom Amplicon Kit is used to create the sample library by hybridizing the probes to the genomic DNA for each sample. Once the probes are ligated to the genomic DNA, creating the amplicon, the TruSeq Amplicon Index Kit is then used to attach sample specific indices and sequencing primers by PCR to each amplicon. The multiple amplicons for each sample are then normalized, pooled together, and sequenced on the Illumina HiSeq system. The paired-end read sequencing data is then exported and data analysis is performed. The sample specific indices added to each amplicon allows for sample identification during data analysis. This technique of targeted resequencing can be used to isolate, sequence, and identify the regions of interest of Fc receptor genes, including the FcRlllb NA1/NA2 polymorphism.

### V. Glycophenotypes of FcγRIIIb NA1 and NA2

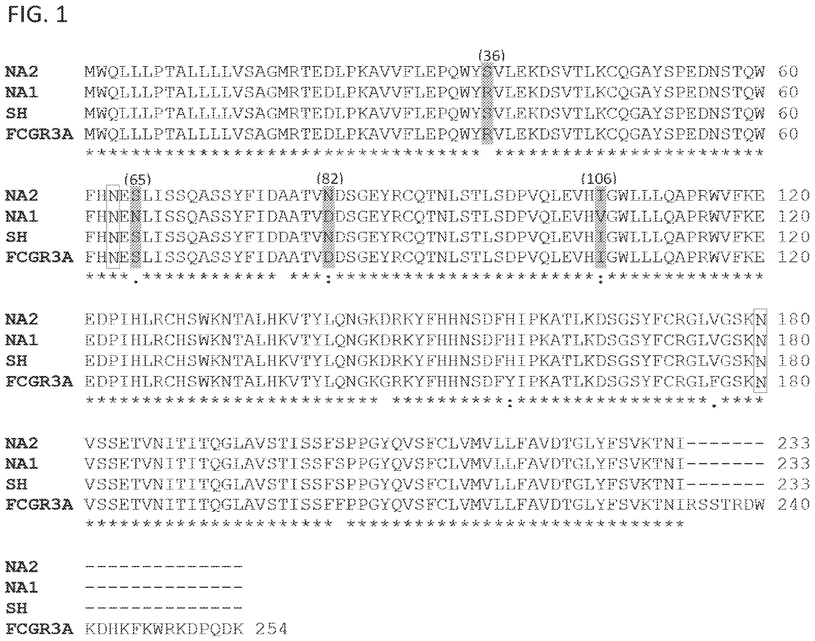

FcγRIIIb NA1 and NA2 display different N-linked glycosylation profiles at amino acid residues N63 and N180 (FIG. 1), which are referred to as N45 and N162 by convention in the literature and are thus referred to as such in the present disclosure. FcγRIIIb NA2 is defined by the presence of high mannose type at N45 and >biantennary at N162, while FcγRIIIb NA1 is defined by the absence of glycans at N45 (through a S47N mutation) and smaller glycans at N162. The glycosylation pattern of FcγRIIIb NA2 is also referred to as the FcγRIIIb NA2 glycophenotype, and the glycosylation pattern of FcγRIIIb NA1 is also referred to as the FcγRIIIb NA1 glycophenotype.

In the present disclosure, it was surprisingly found that the different glycophenotypes of FcγRIIIb are associated with response or non-response to anti-TNF therapy. Thus, methods of treating a subject having an autoimmune disease and/or inflammation include determining the glycophenotype of FcyRlllb in a biological sample from a subject. If the FcγRIIIb NA1 glycophenotype is present in the biological sample, the subject is administered an Fc-activity modulating agent or other non-anti-TNF agent for treating the disease. If the FcγRIIIb NA2 glycophenotype is present in the biological sample, the subject is administered an anti-TNFa agent. The disclosure also provides methods of predicting the responsiveness of a subject to an anti-TNFa treatment, in which the presence of the FcγRIIIb NA1 glycophenotype in a biological sample from the subject is indicative that the subject may not respond to the anti-TNFa treatment.

Examples and figures of the disclosure further demonstrate the association between the glycophenotypes of FcγRIIIb and the responsiveness of subjects having rheumatoid arthritis to an anti-TNFα treatment.

### VI. Detection of Glycopeptides

Glycans of polypeptides can be evaluated using any methods known in the art. Parameters of glycans that may be evaluated include, but are not limited to, detection of N-linked glycan, glycan identification and characterization, glycoform detection, percent glycosylation, and/or aglycosyl. One method of detecting and evaluating glycans is to use fluorescence-activated cell sorting (FACS) (see, e.g., Nagarajan et al 1995 J. Biol. Chem. 270:25762), immunoprecipitation, or differential mobility on SDS-PAGE (see, e.g., Huizinga et al 1990 Blood 75:213). For example, a sandwich ELISA assay can be used to capture FcRlllb from a cell lysate using an anti-Fclllb antibody, followed by detection by another anti-CD16 (FcRIII) antibody (e.g., labeled with a fluor or detected with a labeled secondary antibody). Alternatively a reverse sandwich ELISA could be employed, capturing with the non-interfering anti-CD16 antibody and detecting with an antibody specific for FcRlllb.

The NA1 and NA2 FcyRlllb levels can also be measured by a lectin microtiter ELISA as described in Srinivasan et al. 2015 (Journal of Biomolecular Screening Apr7 epub. PMID:
25851037). This "customizable" ELISA juxtaposes readouts from multiple lectins, exploiting the specificity of different lectin-glycan interactions to obtain quantitative information on protein glycosylation using apposing readouts from a pair of lectins, thus providing an easy approach for attaching proteins of interest, in this case the Fc receptor, on microtiter plates, and glycophenotyping by a single lectin or combination of lectins focusing on the specific glycoform subsets. The mentioned ELISA format involves locking the spatial presentation of the lectins in a multimeric fashion by precomplexing biotinylated lectins to neutravidin to overcome the weak lectin-glycan interaction. The use of neutravidin also precludes the engagement of lectins to epitopes in the complexed state, allowing an unbiased signal generated exclusively due to recognition of glycan on the protein analyte.

Based on prevalent literature on the structural epitopes recognized by lectins and their binding specificities, a panel of lectins can be chosen. In one example, the lectins *Erythrina cristagalli* lectin (ECL), *Ricinus communis* agglutinin (RCA), *Canavalia ensiformis* (ConA), and *Galanthus navali* lectin (GNA) are used to recognize glycan motifs. *Sambucus nigra* agglutinin (SNA) and *Maackia amurensis* lectin (MAL) can be used to discriminate between the 2,6 and 2,3 glycosidic linkages, respectively. Results from the ECL/RCA ELISA when juxtaposed with readings from a SNA/MAL ELISA can be used to inform the levels of the biantennary and triantennary sialylation. The lectins ConA and GNA can detect and quantify mannosylation, in order to be able to distinguish between the NA1 and NA2 based on the high mannose content.

Immunoprecipitation of FcγRIIIb from serum using commercial goat polyclonal antibodies (commercially available from R&D Biosystems) can be followed by coating the protein on 384-well Maxisorp Nunc microtiter plates. Alternatively, plates coated with the goat polyclonal Abs can be incubated first with a non-specific blocking buffer, then with either plasma or cell lysates to capture the FcyRlllb. The protein coated plates are washed to remove unbound protein and blocked with PBSAT, 1% bovine serum albumin (BSA) and 0.5% Tween-20 in PBS. The chosen biotinylated lectins are precomplexed with neutravidin-HRP on ice for 20 min and 50 µL of the lectin-neutravidin-HRP complex added to each well; followed by incubation at room temperature for 2 h. Plates are then washed with PBSAT to remove unbound or weakly bound lectins. After unbound lectins are washed off, binding to the plate is visualized after addition of Amplex Red. The obtained relative fluorescence unit (RFU) values can also be plotted against lectin concentration to compare samples with varying glycosylation levels.

Other methods that may be used to evaluate these glycan parameters are described in, e.g., Chen and Flynn, J. Am. Soc. Mass Spectrom., 20:1821-1833, 2009; Dick et al. Biotechnol. Bioeng.,100:1132-1143, 2008; Goetze et al. Glycobiol., 21:949-959, 2011; and Xie et al. mAbs, 2:379-394, 2010. For example, sialylation of glycan compositions (e.g., level of branched glycans that are sialylated on an α1,3 arm and/or an α1,6 arm) can be characterized using anion-exchange chromatography, which is described in, e.g., Ahn et al. J. Chrom. 878:403-408, 2010.

In some instances, glycan structure and composition as described herein are analyzed, for example, by one or more, enzymatic, chromatographic, mass spectrometry (MS), chromatographic followed by MS, electrophoretic methods, electrophoretic methods followed by MS, nuclear magnetic resonance (NMR) methods, and combinations thereof. Exemplary enzymatic methods include contacting a polypeptide preparation with one or more enzymes under conditions and for a time sufficient to release one or more glycan(s) (e.g., one or more exposed glycan(s)). In some instances, the enzymes include(s) PNGase F. Exemplary chromatographic methods include, but are not limited to, Strong Anion Exchange chromatography using Pulsed Amperometric Detection (SAX-PAD), liquid chromatography (LC), high performance liquid chromatography (HPLC), ultra performance liquid chromatography (UPLC), thin layer chromatography (TLC), amide column chromatography, and combinations thereof. Exemplary mass spectrometry (MS) techniques include, but are not limited to, tandem MS, LC-MS, LC-MS/MS, matrix assisted laser desorption ionization mass spectrometry (MALDI-MS), Fourier transform mass spectrometry (FTMS), ion mobility separation with mass spectrometry (IMS-MS), electron transfer dissociation (ETD-MS), and combinations thereof. Exemplary electrophoretic methods include, but are not limited to, capillary electrophoresis (CE), CE-MS, gel electrophoresis, agarose gel electrophoresis, acrylamide gel electrophoresis, SDS-polyacrylamide gel electrophoresis (SDS-PAGE) followed by Western blotting using antibodies that recognize specific glycan structures, and combinations thereof. Exemplary nuclear magnetic resonance (NMR) include, but are not limited to, one-dimensional NMR (1D-NMR), two-dimensional NMR (2D-NMR), correlation spectroscopy magnetic-angle spinning NMR (COSY-NMR), total correlated spectroscopy NMR (TOCSY-NMR), heteronuclear single-quantum coherence NMR (HSQC-NMR), heteronuclear multiple quantum coherence (HMQC-NMR), rotational nuclear overhauser effect spectroscopy NMR (ROESY-NMR), nuclear overhauser effect spectroscopy (NOESY-NMR), and combinations thereof.

### VII. Pharmaceutical Compositions and Preparations

Pharmaceutical compositions used in methods of the disclosure may include an Fc-activity modulating agent, an anti-TNFa agent (e.g., adalimumab, infliximab, etanercept, certolizumab pegol, or golimumab), a biologic therapy that is not an anti-TNFa agent (e.g., abatacept, tocilizumab, or rituximab), and/or a DMARD (e.g., methotrexate, prednisone, hydroxychloroquine, leflunomide, azathioprine, cyclosporine, tofacitinib, cuprimine, auranofin, or minocycline). Depending on the genotypes of the genes encoding FcγRIIIb, FcγRIIa,FcyRllc and/or the glycophenotype of FcγRIIIb identified in a biological sample of a subject having an autoimmune disease, and/or inflammation, the subject may be administered an appropriate Fc-activity modulating agent. In some instances, the subject may be administered an anti-TNFa agent. In some instances, the subject may be administered an anti-TNFa agent in combination with an Fc-activity modulating agent. In some instances, the subject may be administered a biologic therapy that is not an anti-TNFa agent (e.g., abatacept, tocilizumab, and rituximab) in combination with an Fc-activity modulating agent. In some instances, the subject may be administered a DMARD (e.g., methotrexate, prednisone) in combination with an Fc-activity modulating agent and/or anti-TNFa agent.

Additionally, the pharmaceutical compositions may contain one or more pharmaceutically acceptable carriers or excipients, which can be formulated by methods known to those skilled in the art. Acceptable carriers and excipients in the pharmaceutical compositions are nontoxic to recipients at the dosages and concentrations employed. Acceptable carriers and excipients may include buffers, antioxidants, preservatives, polymers, amino acids, and carbohydrates. Pharmaceutical compositions of the disclosure can be administered parenterally in the form of an injectable formulation. Pharmaceutical compositions for injection (i.e., intravenous injection) can be formulated using a sterile solution or any pharmaceutically acceptable liquid as a vehicle.

### VIII. Routes, Dosage, and Administration

Pharmaceutical compositions used in methods of the disclosure may be formulated for intravenous administration, parenteral administration, subcutaneous administration, intramuscular administration, intra-arterial administration, intrathecal administration, or intraperitoneal administration. The pharmaceutical composition may also be formulated for, or administered via, oral, nasal, spray, aerosol, rectal, or vaginal administration. For injectable formulations, various effective pharmaceutical carriers are known in the art.

The dosage of the pharmaceutical compositions of the disclosure depends on factors including the route of administration, the disease to be treated, and physical characteristics, e.g., age, weight, general health, of the subject. Typically, the amount of an active ingredient contained within a single dose may be an amount that effectively prevents, delays, or treats the disease without inducing significant toxicity. The dosage may be adapted by the physician in accordance with conventional factors such as the extent of the disease and different parameters of the subject.

The pharmaceutical compositions are administered in a manner compatible with the dosage formulation and in such amount as is therapeutically effective to result in an improvement or remediation of the symptoms. The pharmaceutical compositions are administered in a variety of dosage forms, e.g., intravenous dosage forms, subcutaneous dosage forms, and oral dosage forms (e.g., ingestible solutions, drug release capsules). Generally, therapeutic proteins (e.g., an Fc-activity modulating agent) are dosed at 1-100 mg/kg, e.g., 1-50 mg/kg. Pharmaceutical compositions used in methods of the disclosure may be administered to a subject in need thereof, for example, one or more times (e.g., 1-10 times or more) daily, weekly, monthly, biannually, annually, or as medically necessary. Dosages may be provided in either a single or multiple dosage regimens. The timing between administrations may decrease as the medical condition improves or increase as the health of the patient declines.

### IX. Indications

The methods of the disclosure described herein are used to treat a subject having an autoimmune disease and/or inflammation. In some embodiments, an autoimmune disease is a TNF-α mediated disease, which refers to a disease or disorder involving the cytokine TNF-α. TNF-α mediated diseases include, but are not limited to, rheumatoid arthritis (RA), juvenile RA, polyarticular-course juvenile RA, juvenile idiopathic arthritis, psoriasis, psoriatic arthritis, Crohn's Disease, ulcerative colitis, ankylosing spondylitis, plague psoriasis, multiple sclerosis, systemic lupus erythematosus, myasthenia gravis, juvenile onset diabetes, glomerulonephritis, autoimmune thyroiditis, Behcet's disease, graft rejection, graft-versus-host disease, Kawasaki's disease, sarcoidosis, pyoderma gangrenosum, depression, bronchial asthma, diabetes mellitus, malignancies, septic shock, bullous dermatitis, neutrophilic dermatitis, toxic epidermal necrolysis, systemic vasculitis, pyoderma gangrenosum, pustular dermatitis, alcoholic hepatitis, cerebral malaria, hemolytic uremic syndrome, pre-eclampsia, allograft rejection, uveitis, otitis media, snakebite, erythema nodosum, myelodysplastic syndromes, dermatomyositis, polymyositis, immune reconstitution inflammatory syndrome of AIDS patients, systemic sclerosis, IgG4-related disease, and alopecia areata. In some embodiments of any of the described methods of the disclosure, the inflammation is myocarditis.

### EXAMPLES

### Example 1 - Study design

Sixty subjects were selected from the CERTAIN clinical trial for Fcγ receptor IIIb (FcγRIIIb), Ila (FcγRIIa) and Ilc (FcγRIIc) polymorphism analysis. This study recruited rheumatoid arthritis (RA) patients from multiple community and academic clinical centers across North America. Approximately half of the study subjects were responders and half were non-responders to an anti-TNFa treatment by European League Against Rheumatism (EULAR) and American College of Rheumatology (ACR) criteria at three months post treatment. The anti-TNFa treatment includes adalimumab + methotrexate (MTX) or infliximab + MTX. Only subjects with either no other DMARDs (aside from MTX) or stable low dose prednisone were selected. The responders and non-responders were well-matched for demographic and clinical characteristics. The non-responders were also prescreened for plasma drug levels at three months post anti-TNFa treatment to assure that the subjects selected for this study maintained an adequate exposure to the anti-TNFa treatment. Thus, the non-responders' lack of response at three months was not due to poor drug exposure, poor compliance, or anti-drug antibodies. Table 1 below summarizes the numbers and treatments of the responders and non-responders in the study.

**Table 1. Study design**

| Response | Number | Treatment | Co-treatment | **Time** Points |
|---|---|---|---|---|
| Responder | 33 | Anti-TNFa agent (Adalimumab or Infliximab) | MTX +/- stable low dose prednisone (≤10 mg) | Baseline*, 3 months |
| Non-responder | 27 | Anti-TNFa agent (Adalimumab or Infliximab) | MTX +/- stable low dose prednisone (≤10 mg) | Baseline*, 3 months |

| | | | | |
|---|---|---|---|---|
| ^{*}Baseline refers to a time point before starting the anti-TNFa treatment. | | | | |

### Example 2 - Methods of FcγRIIIb and FcγRIIa polymorphism and glycophenotype analysis

Each subject's plasma samples were subjected to targeted proteomics and glycopeptide analysis. Gene expression analysis using Next-Generation sequencing (NGS) was applied to whole blood RNA. Glycopeptide analysis focused on the circulating, soluble forms of FcγRIIIb and FcγRIIa,which are shed from the cell surface of neutrophils and neutrophils/monocytes, respectively.

N-glycopeptides and amino acid changes were quantified following immunoprecipitation of FcγRIIIb and FcγRIIa from serum using commercial goat polyclonal antibodies against these proteins (anti-FcyRII R&D Systems BAF1330, anti-FcγRIII R&D Systems BAF1597). Following immunoprecipitation, the proteins were digested with chymotrypsin or GluC followed by chymotrypsin (GluC/chymotrypsin) to generate peptides and glycopeptides. The specific enzymatic digestion used was tailored for the analysis of different attributes. N-glycosylation at N162 of FcγRIIIb was characterized from the GluC/chymotrypsin digest, while N-glycosylation at N45 of FcγRIIIb and polymorphic variants were characterized from the chymotrypsin digestion. The peptide mixture was analyzed by targeted nLC-MS/MS on a Thermo QExactive mass spectrometer. The isolation width of the quadruple for the mass shown in Tables 2-4 was set at ±1.5 Da.

**Table 2. Mass targeted for characterization of N-glycosylation at N162 of FcγRIIIb**

| **m/z GluC/chymotrypsin digest** | **N162 glycopeptides** | **Proposed structure** |
|---|---|---|
| 989.7 | A1F-1 α2-6 | 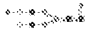 |
| 989.7 | A1F-2 α2-3 | 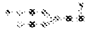 |
| 1209.15 | A2F+LacNAc-1 α2-6,α2-6 | 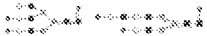 |
| 1209.15 | A2F+LacNAc -2 α2-6,α2-3 | 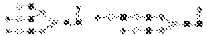 |
| 1111.44 | A1 F+LacNAc-1 α2-6 LacNAc Ext | 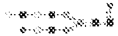 |
| 1111.44 | A1 F+LacNAc-2 α2-6 Triantennary | 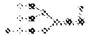 |
| 1111.44 | A1 F+LacNAc-3 α2-3 LacNAc Ext | 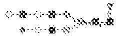 |
| 1111.44 | A1 F+LacNAc-4 α2-3 Triantennary | 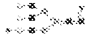 |
| 1086.77 | A2F-1 α2-6,α2-6 | 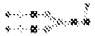 |
| 1086.77 | A2F-2 α2-6,α2-3 | 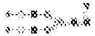 |
| 1086.77 | A2F-3 α2-3,α2-3 | 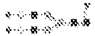 |
| 1038.75 | A1 F+Fuc-1 α2-3 Le^{x/a} | 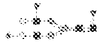 |
| 1038.75 | A1 F+Fuc-2 α2-6 Le^{x/a} | 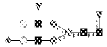 |
| 1038.75 | A1 F+Fuc-3 α2-3 sLe^{x/a} | 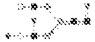 |
| 892.7 | G2F | 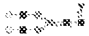 |
| 868 | A1 F-LacNAc-1 α2-6 | 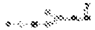 |
| 868 | A1F-LacNAc-2 α2-3 | 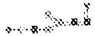 |
| 1160.13 | A1 F+LacNAc+Fuc-1 | 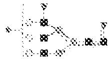 |
| 1160.13 | A1 F+LacNAc+Fuc-2 α2-6/α2-3 Le^{x/a} | 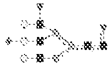 |
| 1160.13 | A1 F+LacNAc+Fuc-3 α2-6/α2-3 sLe^{x/a} | 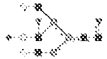 |
| 1160.13 | A1F+LacNAc+Fuc-4 | 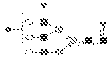 |
| 1135.45 | A2F+Fuc-1 α2-6/α2-3 | 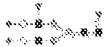 |
| 1135.45 | A2F+Fuc-2 α2-3/α2-3 | 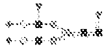 |
| 1257.16 | A2F+LacNAc+Fuc-1 Le^{x/a} | 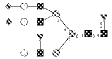 |
| 1257.16 | A2F+LacNAc+Fuc-2 sLe^{x/a} | 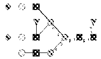 |
| 941.1 | G2F+Fuc | 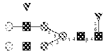 |
| 941.1 | A1 | 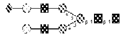 |

**Table 3. Mass targeted for characterization of N-glycosylation at N45 of FcγRIIIb**

| **m/z Chymotrypsin digest** | **N45 glycopeptides** | **Proposed Structure** |
|---|---|---|
| 1394.1 | HM5 | 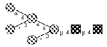 |
| 1475.1 | HM6 | 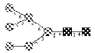 |
| 1556.1 | HM7-1 | 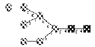 |
| 1556.1 | HM7-2 | 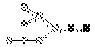 |
| 1637.1 | HM8 | 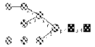 |
| 1718.2 | HM9 | 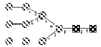 |
| 1143.5 | 3,5,1,1,0-1 α2-6 | 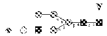 |
| 1143.5 | 3,5,1,1,0-3 α2-3 | 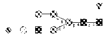 |
| 1094.8 | 3,5,0,1,0-1 α2-6 | 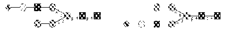 |
| 1094.8 | 3,5,0,1,0-3 α2-3 | 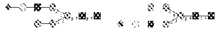 |
| 1148.8 | 3,6,0,1,0-1 α2-6 | 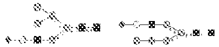 |
| 1148.8 | 3,6,0,1,0-3 α2-3 | 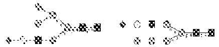 |
| 1040.1 | 3,4,0,1,0-1 α2-6 | 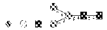 |
| 1040.1 | 3,4,0,1,0-2 α2-3 | 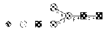 |
| 1088.8 | 3,4,1,1,0-1 α2-6 | 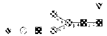 |
| 1088.8 | 3,4,1,1,0-2 α2-3 | 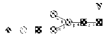 |

In Tables 2 and 3, Ax indicates number terminal sialic acids (on trimannosyl core; A2 indicates disialylated; F linked fucose on antenna; Gx indicates number (x) of b1-4 linked galasose on the antenna; HM indicates high mannose; and HMx indicates number (x) of mannose on core GlcNAcs. In Table 2 Ax refers to the number of terminal sialic acids (i.e A2 has two terminal sialic acids), α2-x refers to the sialic acid linkage, F refers to core fucose (i.e on the the chitobiose core), +Fuc refers to antennary fucose which were known is further classified as Le^{x/a} or sLe^{x/a} to differentiate structures with the Fucose on the sialylated antenna from structures with Fucose on the non-sialylated antenna, structures with +LacNAc are species with greater than 2 antennae, -LacNAc refers to structures with fewer than 2 antennae. Gx refers to non-sialylated structures. In table 3 HMx refers to the number of mannose residues on the GlcNAc core, the numbering of the hybrid species is as follows (HexNAc,Hexose,Fucose,NeuAc,NeuGc) and as in table 2 α2-x refers to the sialic acid linkage.

**Table 4. Mass targeted for characterization of known polymorphic variants for FcγRIIa and FcγRIIIb**

| **m/z Chymotrypsin digest** | **Marker** | **Sequence** |
|---|---|---|
| 701.31 | FcyRIIIb NA1/NA2 D->N65 | FIDA(61)ATVN/D(65)DSGEY |
| 798.87 | FcyRIIIb NA1 N45 | FHN(45)EN(47)LISSQASSY |
| 723.8 | FcyRIIIb SH D61 | FIDD(61)ATVN(65)DSGEY |
| 683.3 | FcyRIIIb NA1 V89 | SDPVQLEVHV(89)GW |
| 690.35 | FcyRIIIb NA2 I89 | SDPVQLEVHI(89)GW |
| 408.7 | FcyRIIa H133 | SH(133)LDPTF |
| 418.2 | FcyRIIa R133 | SR(133)LDPTF |

Amino acid positions 45, 47, 61, 65, and 89 in Table 4 correspond to amino acid positions 63, 65, 78, 82 and 106 in FIG.1, respectively.

Sequence variants in FcγRIIIb at the protein level were detected and quantified using targeted proteomics. Specifically, the R36 and S36 variants associated with the FcγRIIIb NA1 and NA2 haplotypes, respectively, were monitored by this method. A list of peptides monitored by targeted proteomics is provided in Table 5. Non-variant peptides from both FcγRIIIa and FcγRIIIb were also monitored. Plasma samples were depleted of the 14 most abundant proteins and digested with trypsin. Targeted proteomics was performed on the digests using an Orbitrap-Velos mass spectrometer operating in pseudo-multiple reaction monitoring (MRM) mode. Each peptide precursor ion was isolated using a 3 Th isolation window. Extracted ion chromatograms for 5-6 product ions were obtained for all precursors. The summed area of these product ions was used to provide a relative quantitative estimate of each peptide. Areas were normalized to a housekeeping protein, complement factor H (CFH), which had been previously determined to have minimal variability across plasma samples.

**Table 5. Peptides monitored by targeted proteomics**

| Peptide | Sequence | **m/z (Th)** | Charge state |
|---|---|---|---|
| S36 | AVVFLEPQWYSVLEK | 904.49 | +2 |
| R36 | AVVFLEPQWYR | 704.37 | +2 |
| FcγRIIIa non-variant | YFHHNSDFYIPK | 523.25 | +3 |
| FcγRIIIb non-variant | YFHHNSDFHIPK | 514.58 | +3 |

### Example 3 -Results of FcγRIIIb and FcγRIIa polymorphism and glycophenotype analysis

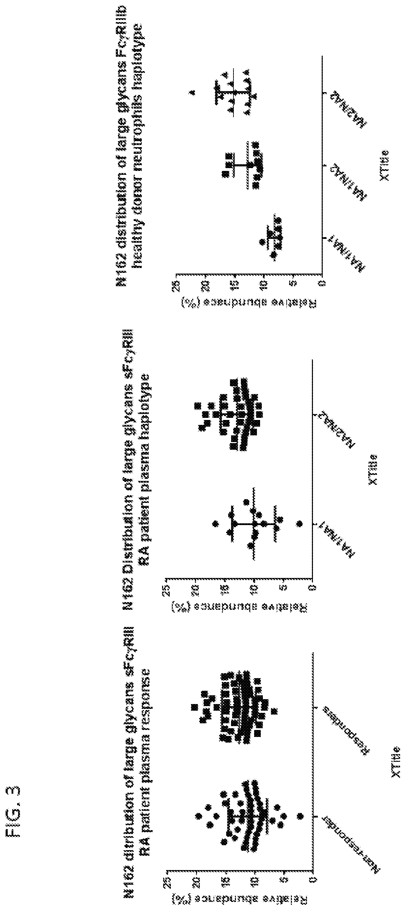

Using the analytical methods described in Example 2, we were able to identify all sites of allelic variation in these two Fcγ receptors, as well as the glycosylation at N45 and N162 of FcγRIIIb. FcγRIIIb has two polymorphic variants named NA1 and NA2, which differ at four amino acid positions: NA1 (R₃₆ N₆₅ D₈₂ V₁₀₆) and NA2 (S₃₆ S₆₅ Ns₂ I₁₀₆) (FIG. 1). We found that the glycosylation profile at N63 and N180 on FcγRIIIb differed in NA1 and NA2 variants both in soluble FcγRIII (sFcγRIII) isolated from patient plasma as well as in soluble FcγRIIIb (sFcγRIIIb) from neutrophils of healthy donors (FIG. 3). These glycans, which occur at positions N63 and N180 as numbered in the sequence alignment shown in FIG. 1, are referred to as N45 and N162 by convention in the literature and are thus referred to as such in the present disclosure.

### Example 4 - Haplotype-specific glycosylation

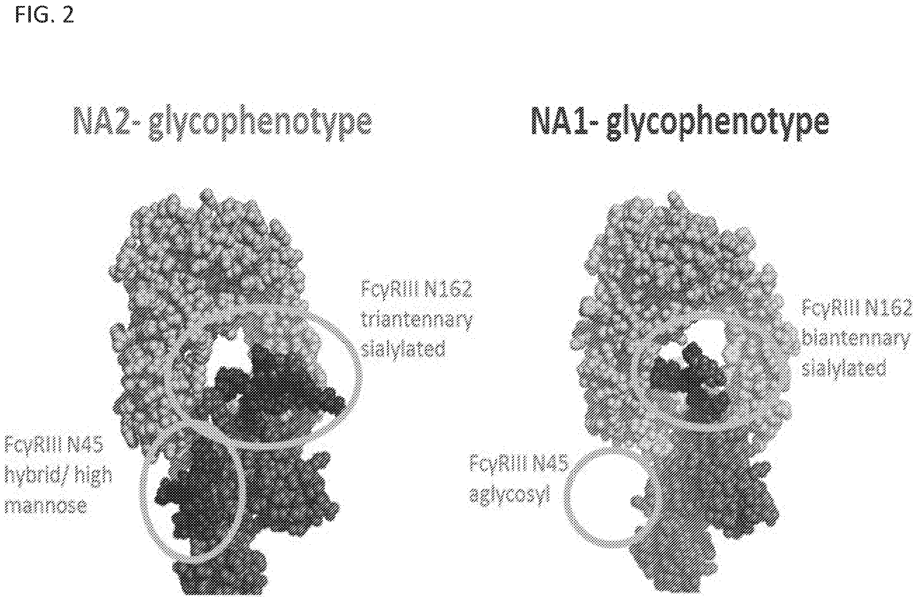

The glycophenotypes of FcγRIIIb NA2 and NA1 at N45 and N162 are haplotype-specific. FIG. 2 shows the haplotype-specific glycosylation at N45 and N162 of FcγRIIIb NA2 and NA1 in molecular models. For FcγRIIIb NA2, N162 appears to be triantennary sialylated and N45 appears to be glycosylated with high mannose. This glycosylation profile of FcγRIIIb NA2 is also referred to as the FcγRIIIb NA2 glycophenotype. For FcγRIIIb NA1, N162 appears to be biantennary sialylated and N45 appears to be aglycosylated. This glycosylation profile of FcγRIIIb NA1 is also referred to as the FcγRIIIb NA1 glycophenotype. FIG. 3 further shows the influence of haploptype on the glycosylation of N162. FIG. 3 (left) shows that responders to the anti-TNFa agent + MTX treatment have a higher relative abundance of highly branched (> biantennary (i.e., triantennary)) N-glycans at N162 than non-responders do. Moreover, subjects having RA who are FcγRIIIb NA2 homozygous (NA2/NA2) appeared to have a higher relative abundance of highly branched (> biantennary (i.e., triantennary)) N-glycans at N162 than subjects who are NA1 homozygous (NA1/NA1) (FIG. 3 (middle)). The relative abundance of glycosylated N162 also differed between FcγRIIIb NA1 and NA2 from neutrophils of healthy donors. Glycosylation at N162 of FcγRIIIb isolated from the neutrophils of healthy donors shows the same association between N162 glycosylation and haplotypes. Furthermore, healthy donors who are FcγRIIIb heterozygous (NA1/NA2) displayed an intermediate relative abundance of highly branched (> biantennary (i.e., triantennary)) N-glycans at N162 compared to healthy donors who are NA1/NA1 and NA2/NA2 (FIG. 3 (right)). These results point to an effect of FcγRIIIb haplotype on glycosylation. This effect may be important in the affinity or kinetics of the interaction between antibodies and Fcγ receptors.

### Example 5 - Association between treatment responsiveness, FcγRIIIb genotype, and FcγRIIIb glycophenotype

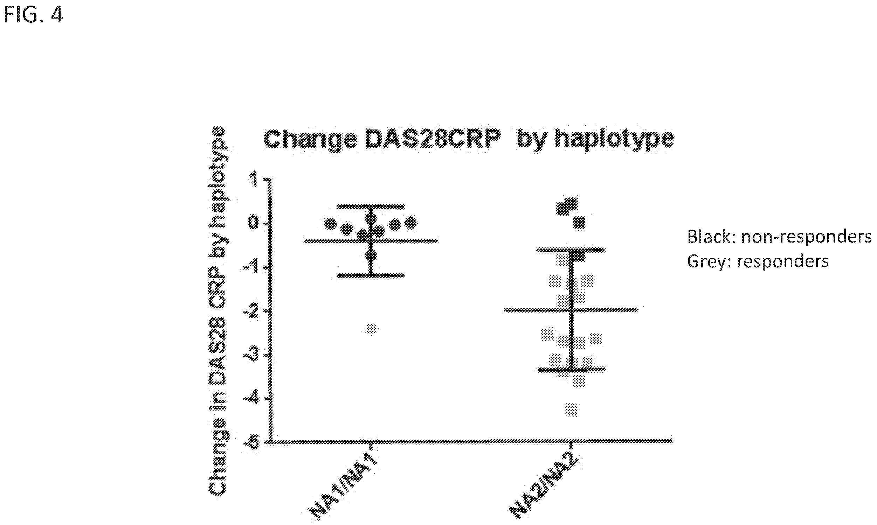

Our results showed that the glycosylation and protein sequences of FcγRIIIb NA2 and NA1 did not change between baseline and three months post anti-TNFa agent + MTX treatment, which suggests that anti-TNFa agent + MTX treatment or variation in disease score did not alter the glycoprotein phenotype of these receptors. However, we observed a strong association among response to anti-TNFa agent + MTX treatment at three months, FcγRIIIb genotype, and FcγRIIIb glycophenotype (Table 6A and FIG. 4). Table 6A shows the number of responders and non-responders by their FcγRIIIb glycophenotype and the strong statistically-significant bias towards good response in FcγRIIIb NA2/NA2 homozygous subjects and non-response in FcγRIIIb NA1/NA1 homozygous subjects. This finding is also illustrated in FIG. 4, which shows that change in disease severity (which is measured by the change in 28-joint Disease Activity Score using C-reactive protein value (DAS28 CRP score)) after anti-TNFa agent + MTX treatment in subjects who are FcγRIIIb NA1 homozygous (NA1/NA1) or NA2 (NA2/NA2). Most of NA2/NA2 homozygous subjects appeared to have good response to anti-TNFa agent + MTX treatment as shown by the negative DAS28 CRP score.

**Table 6A**

| **FcγRIIIb variant** | **NA1/NA1** | **NA2/NA2** | **NA1/NA2** |
|---|---|---|---|
| Responder | 1 | 16 | 15 |
| Non-responder | 8 | 4 | 15 |

After the initial study, we further expanded the total number of subjects with rheumatoid arthritis (RA) analyzed by our methodology to a total of approximately 145. Based on the results of this analysis we obtained the data shown in Table 6B below:

**Table 6B**

| **FcγRIIIb variant** | **NA1/NA1** | **NA2/NA2** | **NA1/NA2** |
|---|---|---|---|
| Responder | 6 | 29 | 32 |
| Non-responder | 16 | 22 | 40 |

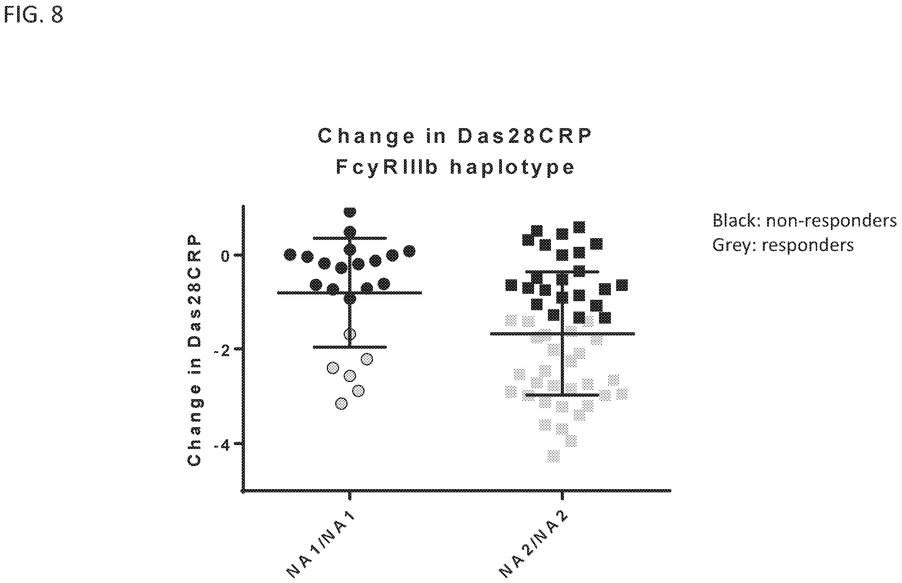

These results expand our number of NA1/NA1 RA subjects from the initial study described in Example 1. We continue to observe that a greater number of NA1/NA1 subjects tend to be non-responders to anti-TNFα agent + MTX treatment (FIG. 8). (Note: these results include subjects with low drug levels as well).

### Example 6 - Glycophenotypes in NA1/NA2 heterozygous subjects

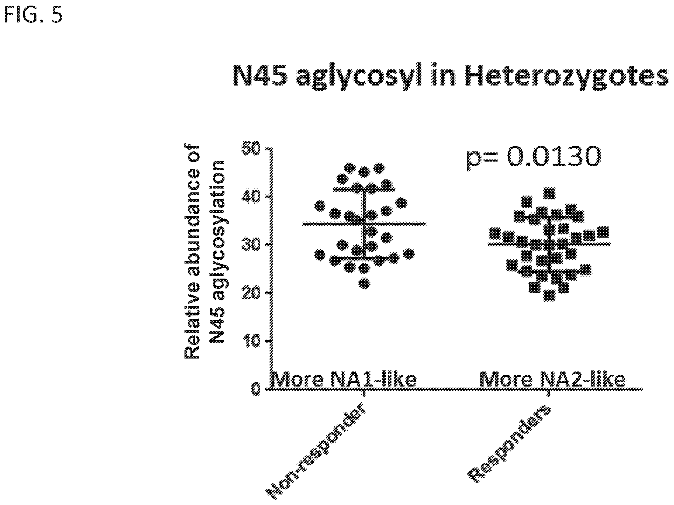

As would be expected by the frequency of the NA1 and NA2 haplotypes in the general population, the NA1/NA2 heterozygotes are the largest subgroup. Non-responders and responders are equally represented in these heterozygotes. However, FIG. 5 shows that the relative expression of glycopeptides in this group is biased towards the NA1- or NA2-like glycophenotypes depending on the individual subject's response at three months post anti-TNFa agent + MTX treatment. In FIG. 5, increased N45-aglycosylation in NA1/NA2 heterozygous non-responders indicates that the N45 glycosylation in these subjects is more similar to the NA1-glycophenotype (see FIG. 2). Similarly, N45 aglycosylation in NA1/NA2 heterozygous responders indicates that the N45 glycosylation in these subjects is more similar to the NA2-glycophenotype. Thus, as is shown in FIG. 5, although the NA1/NA2 heterozygotes share both polymorphic variants, glycophenotypes of their circulating FcγRIIIb appear to be more NA1- or NA2-like depending on their treatment response status.

### Example 7 - Association between treatment responsiveness, FcγRIIIb genotype, and FcγRIIa H/R131 polymorphism

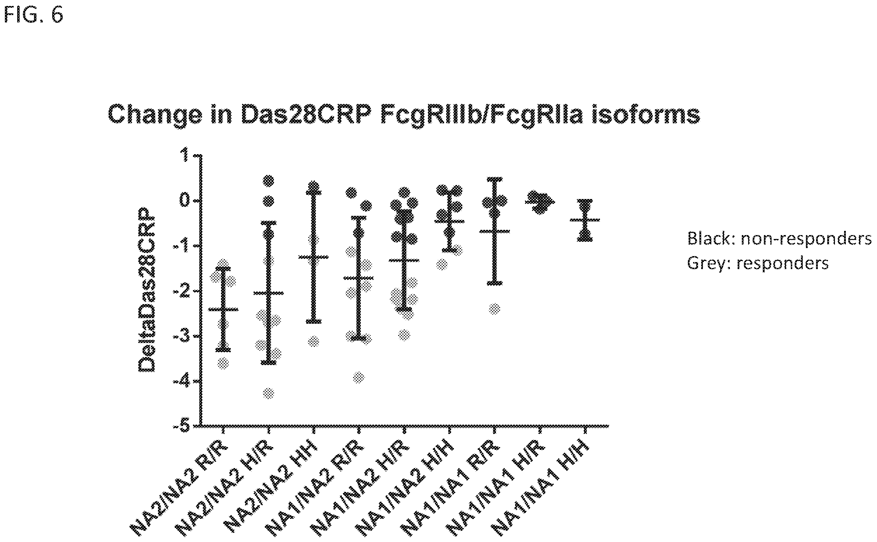

While the FcγRIIIb genotype showed a significant association with anti-TNFa agent + MTX treatment response status, we found an even stronger association when the FcγRIIa H/R131 polymorphism is considered in combination with the FcγRIIIb NA1/NA2 polymorphism (FIG. 6). The combination of FcγRIIIb NA1 homozygous (NA1/NA1) and FcγRIIa H131 homozygous (HH) was significantly associated with non-response. The combination of the mixed haplotypes FcγRIIIb NA1/NA2 (NA1/NA2) and FcγRIIa R/H131 (H/R) showed intermediate distribution of responders and non-responders.

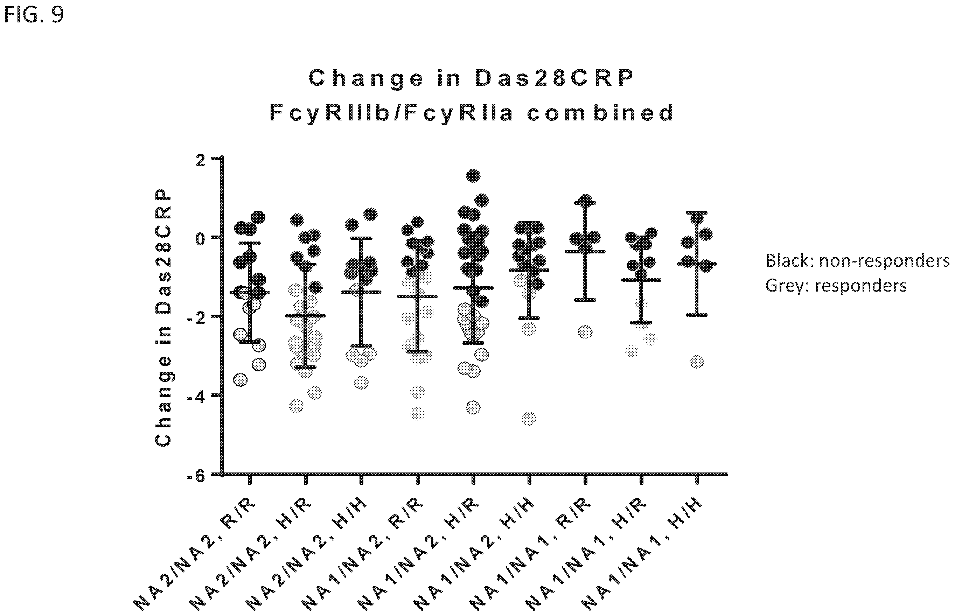

We further included additional subjects to the analysis. The combination of FcγRIIIb NA1 homozygous (NA1/NA1) and FcγRIIa H131 homozygous (H/H) continues to shows a trend towards non-response (FIG. 9). However the combination of FcγRIIIb NA2 homozygous (NA2/NA2) and FcγRIIa R131 homozygous (R/R) does not show a strong association with good response to anti-TNFa agent + MTX treatment (FIG. 9).

### Example 8 - Association between treatment responsiveness, and FcγRIIc genotype

Gene expression analysis using Next-Generation sequencing (NGS) was applied to each subject's whole blood RNA. Total RNA was isolated from whole blood samples stored in PaxGene tubes using PAXgene Blood miRNA Kit. Quality control using the Bioanalyzer RNA kit was performed on the samples to determine total RNA concentration and RNA integrity number (RIN). Concentration of total RNA for all of the samples were normalized and sent to the Whitehead Institute Genome Technology Core lab. A library for each sample was generated using the TruSeq Stranded mRNA Library Prep Kit. Quality control using the Bioanalizer was performed on the library to determine fragment length and uniformity. This was followed by quantification by qPCR. Eleven samples with low RIN scores and/or poor library properties are excluded from further analysis. Sequencing of the samples was performed on the Illumima HiSeq 2500 system generating 40 base read lengths of between 15-110 million 40 base reads per sample. Five runs were performed to sequence all of the samples. This system generates two FASTQ files for each sample, one for the forward paired end read and one for the reverse paired end read from the Illumina solexa pipeline version 1.4 where the basecalls and sequencing quality scores per base read are recorded. Post run quality control reports are generated to monitor general parameters of the sequencing run.

The FASTQ files were aligned to the reference human build GRCh37.p13 and annotated with the USCS genes using the Two-Pass method with the STAR sequence alignment algorithm. The STAR sequence alignment algorithm generates a single binary sequence alignment map (BAM) file containing a binary description of the aligned reads. The quality of the alignments was tested with the RNA-SeQC tool from the Broad Institute. Gene expression levels were obtained from the counts for the reads extracted for each sample BAM file using the featureCounts algorithm. SNP data was extracted from the BAM files for each annotated gene and chromosomal position using samtools and VarScan algorithms which count the number of reads of the reference base and variant base and generate a p-value for the assignment of homozygous reference, homozygous variant or heterozygous to each sample.

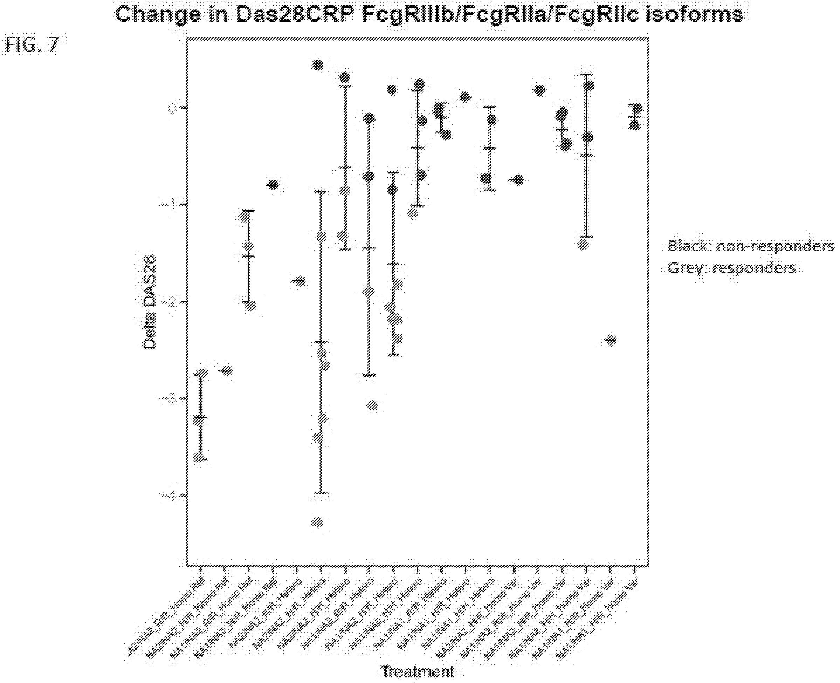

One of the genotypes identified in the analysis is FCGR2C Chr1:161569198 (registered in the NCBI dbSNP as ID rs61801826). This SNP (referred to herein as FcγRIIc variant rs61801826) upon further analysis showed association with response to anti-TNFa treatment. Table 7 shows the number of responders and non-responders by their FcγRIIc genotype and the strong statistically-significant bias towards good response in FcγRIIc homozygous reference subjects and non-response in FcγRIIc homozygous variant subjects (also see FIG. 7).

**Table 7**

| **FcγRIIc variant rs61801826** | **Homozygous Reference** | **Homozygous Variant** | **Heterozygous** |
|---|---|---|---|
| Responder | 8 | 2 | 17 |
| Non-responder | 1 | 10 | 15 |

### Example 9 - FcR SNPS associated with response or non-response to Anti-TNFa treatment

Table 8 contains a list of additional FcyR SNPs that show association with response or non-response to anti-TNFa treatment and the genotype of the patient. The table has the gene name, chromosome number, chromosome position and the dbSNP rs id to additionally identity the SNP. The chromosome position is based on the reference human genome build GRCh37.p13. In addition, columns "Homozygous Reference Predictor", "Homozygous Variant Predictor", and "Heterozygote Predictor" identifies for that genotype whether it is a predictor of response or non-response. Any of the methods described herein can also be used with the SNPs and associated response listed in Table 8.

**Table 8**

| **Gene** | **Chromosome** | **Position** | **dbSNP RSID** | **Homozygous Reference Predictior** | **Homozygous Variant Predictor** | **Heterozygote Predictor** |
|---|---|---|---|---|---|---|
| FCGR2A | chr1 | 161487375 | rs4285675 | Response | | Non-Response |
| FCGR2A | chr1 | 161487543 | rs2085697 | Response | | Non-Response |
| FCGR2C | chr1 | 161569148 | rs422670 | Response | Non-Response | Response |
| FCGR2C | chr1 | 161566260 | rs79246620 | Response | Non-Response | Response |
| FCGR2C | chr1 | 161569419 | rs430178 | Response | | Non-Response |
| FCGR2C | chr1 | 161567927 | rs72700098 | Response | Non-Response | |
| FCGR2C | chr1 | 161567986 | rs76052159 | Response | | Non-Response |
| FCGR2C | chr1 | 161569028 | rs416415 | Response | Non-Response | |
| FCGR2C | chr1 | 161567194 | rs72700096 | Response | Non-Response | Response |
| FCGR2C | chr1 | 161569030 | rs2085696 | Response | Non-Response | |
| FCGR2C | chr1 | 161569518 | rs541523000 | Response | | Non-Response |
| FCGR2C | chr1 | 161570357 | rs1674785 | Response | | Non-Response |
| FCGR3A | chr1 | 161518159 | rs200225944 | Response | Non-Response | |
| FCGR3A | chr1 | 161518214 | rs148181339 | Response | | |
| FCGR3A | chr1 | 161511685 | rs15811 | Response | | |
| FCGR3A | chr1 | 161513118 | rs10800580 | Response | Non-Response | |
| FCGR3B | chr1 | 161599571 | rs2290834 | Response | Non-Response | |
| FCGR3B | chr1 | 161593122 | rs146653557 | Response | Non-Response | |
| FCGR3B | chr1 | 161593880 | rs79636887 | | Non-Response | |
| FCGR3B | chr1 | 161598278 | rs552938096 | Non-Response | Response | |
| FCGR3B | chr1 | 161599693 | rs448740 | | Response | |
| FCGR3B | chr1 | 161597206 | rs72702132 | Response | Non-Response | |
| FCGR3B | chr1 | 161600592 | rs72704050 | Non-Response | Response | |
| FCGR3B | chr1 | 161594100 | rs61803007 | Non-Response | Response | |
| FCGR3B | chr1 | 161596704 | rs2290832 | Non-Response | Response | |
| FCGR3B | chr1 | 161600995 | rs34085961 | Response | Non-Response | |
| FCGR3B | chr1 | 161597821 | rs67295569 | Non-Response | Response | Response |
| FCGR3B | chr1 | 161597264 | rs74127074 | Response | Non-Response | Non-Response |
| FCGR3B | chr1 | 161601173 | rs34322334 | Response | Non-Response | |

## Claims

1. A method of predicting the responsiveness of a patient to an anti-TNFa treatment, said method comprising:
(a) identifying a patient having inflammation or an autoimmune disease;
(b) determining the presence of the FcγRIIc in a biological sample from said patient, and
(c) selecting the patient for treatment with an anti-TNFa agent if the patient lacks the FcγRIIc variant rs61801826.

## Patentansprüche

1. Verfahren zur Vorhersage des Ansprechens eines Patienten auf eine Anti-TNFα-Behandlung, wobei das Verfahren umfasst:
a) Identifizieren eines Patienten mit einer Entzündung oder einer Autoimmunkrankheit;
b) Bestimmen des Vorhandenseins von FcγRIIc in einer biologischen Probe von dem Patienten und
c) Auswählen des Patienten für eine Behandlung mit einem Anti-TNFα-Mittel, wenn dem Patienten die FcγRIIc-Variante rs61801826 fehlt.

## Revendications

1. Procédé de prédiction de la réactivité d'un patient à un traitement anti-TNFα, ledit procédé comprenant :
a) l'identification d'un patient présentant une inflammation ou une maladie auto-immune ;
b) la détermination de la présence du récepteur FcγRIIc dans un échantillon biologique provenant dudit patient, et
c) la sélection du patient pour un traitement par un agent anti-TNFα si le patient n'a pas la variante rs61801826 du récepteur FcγRIIc.
